(19) Europäisches Patentamt
European Patent Office
Office européen des brevets

(11) **EP 2 025 320 A1**

(12) **DEMANDE DE BREVET EUROPEEN**

(43) Date de publication:
 **18.02.2009  Bulletin 2009/08**

(51) Int Cl.:
 *A61K 8/25* (2006.01)      *A61K 8/26* (2006.01)
 *A61K 8/29* (2006.01)      *A61K 8/41* (2006.01)
 *A61Q 3/02* (2006.01)

(21) Numéro de dépôt: 08161339.0

(22) Date de dépôt: **29.07.2008**

(84) Etats contractants désignés:
 **AT BE BG CH CY CZ DE DK EE ES FI FR GB GR HR HU IE IS IT LI LT LU LV MC MT NL NO PL PT RO SE SI SK TR**
 Etats d'extension désignés:
 **AL BA MK RS**

(30) Priorité: **10.08.2007  FR 0705815**
 **10.08.2007  FR 0705814**

(71) Demandeur: **L'Oréal**
 **75008 Paris (FR)**

(72) Inventeurs:
 • **Guerchet, Laurence**
  **94550 CHEVILLY-LARUE (FR)**

 • **Coffey-Dawe, Lizabeth-Anne**
  **93600 AULNAY SOUS BOIS (FR)**
 • **Abergel, Aline**
  **92100 BOULOGNE (FR)**
 • **Ramet, Marc**
  **95100 Argenteuil (FR)**

(74) Mandataire: **Boulard, Denis**
 **L'Oréal**
 **River Plaza - DIPI**
 **25-29 Quai Aulagnier**
 **92665 Asnières-sur-Seine (FR)**

(54)   **Ensemble de conditionnement et d'application d'un vernis à ongles de viscosité élevée**

(57)   La présente invention concerne un ensemble (1) de conditionnement et d'application comportant :
- un récipient (3) contenant une composition de vernis à ongles comprenant un milieu cosmétiquement acceptable et au moins un agent épaississant thixotrope, ladite composition présentant une viscosité à 25°C d'au moins 0,6 Pa.s,
- un applicateur (2) pour prélever le produit contenu dans le récipient et l'appliquer sur les ongles, comportant une lame souple (6) définissant une face applicatrice (15).

Fig.1

**Description**

La présente invention concerne un ensemble de conditionnement et d'application destinés à l'application d'un produit de viscosité élevée sur les ongles. La présente invention concerne également un vernis à ongles de viscosité élevée et de texture gélifiée comprenant des agents de coloration particuliers ainsi qu'un procédé de revêtement des ongles.

La composition ou produit de vernis à ongles, colorée ou transparente, peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles.

Les vernis à ongles conventionnels se présentent sous forme liquide ou fluide et sont généralement conditionnés dans des flacons. Ils comprennent en général des particules solides telles que les pigments, les nacres, les charges, qui sont en dispersion dans le milieu continu aqueux ou le milieu solvant organique de la composition.

Cette forme fluide impose une bonne dispersion des pigments de manière à préserver l'homogenéité de la couleur du vernis liquide ainsi que du film de vernis une fois appliqué sur les ongles.

Or ces particules ont tendance à sédimenter au cours du temps, du fait de leur densité qui est supérieure à celle du milieu continu dans lequel elles sont dispersées. Cette sédimentation se traduit par une modification de l'aspect macroscopique de la composition, et en particulier, dans le cas de vernis à ongles colorés, par une hétérogénéité de la couleur du vernis. L'incorporation de ce type de particules, en particulier des nacres et des pigments magnétiques, est donc limitée (de l'ordre de 1 à 2% maximum selon le type de particules) dans les vernis à ongles conventionnels sous forme liquide.

On cherche donc à obtenir des vernis à ongles présentant une bonne dispersion des particules, en particulier des pigments, et donc une bonne stabilité et une bonne homogénéité de la couleur dans le temps, ainsi qu'un film déposé sur les ongles ayant une couvrance satisfaisante. Le vernis permet également l'obtention d'effets coloriels satisfaisants, non limités.

En outre d'un point de vue pratique, il serait intéressant de disposer de vernis présentant de nouvelles textures qui soient faciles à manipuler (sans problèmes de renversement ou de goutte).

La demanderesse a constaté que ces avantages pouvaient être obtenus par la mise en oeuvre d'une composition de vernis à ongles se présentant sous forme non liquide, de haute viscosité et de texture gélifiée permettant une dispersion homogène des pigments. Selon une alternative, les pigments sont présents en une teneur supérieure ou égale à 2% en poids de la composition.

De plus cette texture gélifiée permet une meilleure organisation et orientation des particules colorantes (en particulier des nacres) dans la composition lors de son application sur les ongles puis lors du séchage du film de vernis, permettant ainsi d'obtenir un effet coloriel et une brillance supérieurs aux films issus de vernis à ongles fluides conventionnels dans lesquels les particules colorantes ne suivent pas une orientation préférentielle.

En outre, cette composition, contrairement aux vernis à ongles conventionnels, ne coule pas, ne goutte pas et permet d'obtenir, après application sur les ongles, un film qui sèche rapidement tout en étant homogène et lisse; et qui présente de bonnes propriétés de tenue et de brillance.

D'autre part, les vernis à ongles et produits de soins pour les ongles conventionnels sont généralement appliqués à l'aide de pinceaux réalisés en agrafant une touffe de poils à l'extrémité d'une tige. De tels pinceaux offrent le plus souvent satisfaction pour les produits à consistance liquide.

Par contre, pour des produits plus visqueux, les pinceaux conventionnels ne donnent pas de bons résultats car les poils forment à la surface du produit des stries qui persistent au séchage.

Il existe un besoin pour bénéficier de vernis présentant de nouvelles textures de haute viscosité et de texture gélifiée qui soient faciles à manipuler (sans problèmes de renversement ou de goutte), présentant une bonne dispersion des particules, et donc une bonne stabilité et une bonne homogénéité de la couleur dans le temps, ainsi que d'un applicateur convenant plus particulièrement à l'application dudit vernis présentant une texture de gel.

Selon un premier de ses aspects, l'invention a ainsi pour objet un ensemble de conditionnement et d'application comportant :

-    un récipient (3) contenant une composition (ou produit) de vernis à ongles comprenant un milieu cosmétiquement acceptable et au moins un agent épaississant thixotrope, ladite composition présentant une viscosité à 25˚C d'au moins 0,6 Pa.s,
-    un applicateur (2) pour prélever le produit contenu dans le récipient et l'appliquer sur les ongles, comportant une lame souple (6) définissant une face applicatrice (15).

Selon un autre aspect, la présente invention a pour objet une composition de vernis à ongles comprenant un milieu cosmétiquement acceptable, au moins un agent épaississant thixotrope, au moins un agent de coloration choisi parmi les corps magnétiques, les pigments diffractants, les pigments interférentiels, les particules réfléchissantes et leurs mélanges, ledit agent de coloration étant présent en une teneur supérieure ou égale à 2% en poids par rapport au poids total de la composition, ladite composition présentant une viscosité à 25˚C d'au moins 0,6 Pa.s.

En particulier, la nature et/ou la quantité dudit agent épaississant sont telle(s) que, en réponse à une action non chimique, notamment mécanique, préalablement ou simultanément à l'application de la composition sur les ongles, la viscosité de la composition peut être abaissée de manière réversible à une valeur n'excédant pas 0,4 Pa.s, de préférence à une valeur n'excédant pas 0,3 Pa.s.

Selon un autre aspect, l'invention a pour objet un ensemble de conditionnement et d'application comportant :

- un récipient (3) contenant une composition (ou produit) de vernis à ongles comprenant un milieu cosmétiquement acceptable, au moins un agent épaississant thixotrope, au moins un agent de coloration choisi parmi les corps magnétiques, les pigments diffractants, les pigments interférentiels, les particules réfléchissantes et leurs mélanges, ledit agent de coloration étant présent en une teneur supérieure ou égale à 2% en poids par rapport au poids total de la composition, ladite composition présentant une viscosité à 25˚C d'au moins 0,6 Pa.s, et
- un applicateur (2) pour prélever le produit contenu dans le récipient et l'appliquer sur les ongles, comportant une lame souple (6) définissant une face applicatrice (15).

De préférence, les compositions selon l'invention sont exemptes de camphre. Par « exempte », on entend que la composition ne contient pas du tout de camphre, soit une quantité de 0% en poids par rapport au poids total de la composition.

Mesure de la viscosité

La viscosité de la composition est mesurée à 25˚C à l'aide d'un Rhéomat 180 (Société LAMY) équipé d'un mobile MS-R1, MS-R2, MS-R3, MS-R4 ou MS-R5 choisi en fonction de la consistance de la composition, tournant à une vitesse de rotation de 200 t.min-1. La mesure est prise après 10 min de rotation. Les mesures de viscosité sont réalisées au maximum 1 semaine après fabrication.

Le vernis à ongles de l'invention peut présenter une viscosité allant de 0,6 à 20 Pa.s, de préférence de 0,7 à 15 Pa.s et, mieux de 0,75 à 10 Pa.s.

L'invention a également pour objet un ensemble (1) de conditionnement et d'application comportant :

- un récipient (3) contenant une composition de vernis à ongles comprenant un milieu cosmétiquement acceptable et au moins un agent épaississant thixotrope, ladite composition présentant un caractère thixotropique,
- un applicateur (2) pour prélever le produit contenu dans le récipient et l'appliquer sur les ongles, comportant une lame souple (6) définissant une face applicatrice (15).

L'invention a également pour objet un ensemble (1) de conditionnement et d'application comportant :

- un récipient (3) contenant une composition de vernis à ongles comprenant un milieu cosmétiquement acceptable et au moins un agent épaississant thixotrope, ladite composition présentant un module de rigidité plateau Gp supérieur à 100 Pa,
- un applicateur (2) pour prélever le produit contenu dans le récipient et l'appliquer sur les ongles, comportant une lame souple (6) définissant une face applicatrice (15).

L'invention a encore pour objet un procédé cosmétique de maquillage des ongles consistant à appliquer sur les ongles une composition telle que définie plus haut.

L'invention a aussi pour objet l'utilisation d'une composition telle que définie ci-dessus pour obtenir un film de vernis déposé sur les ongles, homogène et présentant de bonnes propriétés de brillance et/ou une bonne couvrance.

Par milieu cosmétiquement acceptable, on entend un milieu non toxique et susceptible d'être appliqué sur les matières kératiniques d'êtres humains, en particulier les ongles.

La composition selon l'invention présente avantageusement un caractère thixotrope.

Par composition ayant un caractère thixotrope, ou composition thixotrope, on entend, au sens de l'invention, une composition structurée qui fluidifie (notamment sa viscosité diminue) lorsqu'on lui applique une action non chimique, en particulier mécanique, et qui récupère toute ou partie de sa viscosité initiale après un temps de repos suffisant qui peut être plus ou moins long, à température ambiante.

En particulier, la composition a les propriétés suivantes :

- la composition a un caractère rhéofluidifiant, c'est-à-dire que la viscosité de la composition diminue lorsque l'on applique à la composition des cisaillements croissants ;

- la composition après l'application d'un cisaillement intense se fluidifie (notamment sa viscosité diminue). La viscosité, la consistance et l'élasticité de la composition, après sa destructuration, en particulier après un temps de repos d'une minute après avoir appliqué le cisaillement, sont inférieures à celles de la composition avant l'application du cisaillement intense ;
- la composition régénère partiellement ou totalement sa structure initiale après un temps de repos suffisant et la restructuration de la composition est retardée dans le temps. La restructuration de la composition ne se produit donc pas instantanément mais de façon différée dans le temps.

Une définition de composition thixotrope est notamment indiquée dans l'ouvrage "Comprendre la rhéologie - De la circulation du sang à la prise du béton" de P. Coussot et J.L. Grossiord, EDP Science, 2002, pages 16 et 17.
Le comportement thixotrope de la composition peut notamment être caractérisé via les mesures de viscosité de la composition sous faible vitesse cisaillement puis sous forte vitesse de cisaillement, comme décrit ci-après :

Ces mesures sont effectuées sur un rhéomètre à contrainte imposée, Haake RheoStress® RS 600 de la société ThermoRhéo, équipé d'un bain thermostaté et d'un mobile en acier inoxydable à géométrie cône/plan, de diamètre 35 mm et d'angle 2˚, avec un entrefer de 0,104 mm. Les 2 surfaces sont « sablées » pour limiter les phénomènes de glissement aux parois. Un dispositif anti-évaporation (cloche à solvants) est utilisé.

Les mesures sont effectuées à 20˚C $\pm$ 1 ˚C.
Dans un premier temps, l'échantillon est mis en température à 20˚C $\pm$ 1 ˚C pendant 300 secondes (sans aucun cisaillement appliqué).

a) On applique des contraintes croissantes à l'échantillon en partant d'une contrainte initiale de 0 Pa pour arriver à une contrainte finale de 500 Pa, les contraintes n'étant appliquées qu'une seule fois (ce qui correspond à une rampe en cisaillement allant de $10^{-1}$ $s^{-1}$ à 400 $s^{-1}$). On trace alors l'évolution de la viscosité $\eta$ en fonction du gradient de cisaillement $\dot{\gamma}$. On prend la mesure sur 40 points répartis de façon logarithmique.
On attend l'obtention d'une valeur stable entre chaque contrainte, le temps d'attente entre chaque contrainte étant de 30 s.

b) On destructure ensuite la composition en lui appliquant un cisaillement continu correspondant à une contrainte de 500Pa (correspondant à un cisaillement de 400 $s^{-1}$), pendant 30 secondes.

c) On applique à l'échantillon des contraintes décroissantes en partant d'une contrainte initiale de 500 Pa pour arriver à une contrainte finale de 0 Pa, les contraintes n'étant appliquées qu'une seule fois.
On prend la mesure sur 40 points répartis de façon logarithmique.
On attend l'obtention d'une valeur stable entre chaque contrainte, le temps d'attente entre chaque contrainte étant de 30 s.
La gamme de gradient de cisaillement va de 400 $s^{-1}$ à $10^{-3}$ $s^{-1}$. Dans la gamme considérée, la valeur maximale de 400 $s^{-1}$ doit être prise en compte avec une incertitude de mesure de $\pm$ 10 $s^{-1}$
On trace alors l'évolution de la viscosité $\eta$ en fonction du gradient de cisaillement $\dot{\gamma}$

d) L'échantillon est ensuite soumis à une contrainte imposée de 10 Pa pendant 1000 s, et on mesure l'évolution de la viscosité en fonction du temps.

e) On applique à nouveau des contraintes croissantes à l'échantillon en partant d'une contrainte initiale de 0 Pa pour arriver à une contrainte finale de 500 Pa, les contraintes n'étant appliquées qu'une seule fois (ce qui correspond à un gradient de cisaillement allant de $10^{-2}$ $s^{-1}$ à 300 $s^{-1}$).

L'analyse des résultats se fait au travers de la représentation graphique de l'évolution de la viscosité, notée $\eta$, en fonction du gradient de cisaillement, noté $\dot{\gamma}$. La viscosité $\eta$, le gradient de cisaillement $\dot{\gamma}$ et la contrainte imposée $\tau$ étant reliés entre eux par la relation

$$\dot{\gamma} = \frac{\tau}{\eta}$$

La composition, est telle qu'elle présente une viscosité, telle que mesurée au cours de l'étape e), à une vitesse de cisaillement de $4.10^{-2}$ s$^{-1}$, allant de $10^2$ à $10^4$ Pa.s, mieux de $5.10^2$ à $5.10^3$ Pa.s, encore mieux de 600 à 4000 Pa.s.

Le caractère rhéofluidifiant de la composition est notamment caractérisé par une différence (viscosité mesurée à l'étape e), à une vitesse de cisaillement de 100 s$^{-1}$ - viscosité mesurée mesurée à l'étape e), à une vitesse de cisaillement de $4.10^{-2}$ s$^{-1}$), allant de 10 à $10^5$ Pa.s, mieux de $10^2$ à $10^4$ Pa.s.

Le comportement thixotrope de la composition est caractérisé par une différence de viscosité mesurée à une vitesse de cisaillement de 1 s$^{-1}$ entre l'étape c) et l'étape e) (viscosité mesurée au cours de l'étape c) à une vitesse de cisaillement de 1 s$^{-1}$ - viscosité mesurée au cours de l'étape e) d'au moins 1 Pa .s, de préférence d'au moins 10 Pa.s, mieux d'au moins 20 Pa.s, encore mieux d'au moins 30 Pa.s, préférentiellement d'au moins 40 Pa.s.

En particulier, la différence de viscosité mesuré à une vitesse de cisaillement de 1 s$^{-1}$ entre l'étape c) et l'étape e) soit la différence (viscosité mesurée au cours de l'étape c) à une vitesse de cisaillement de 1 s$^{-1}$ - viscosité mesurée au cours de l'étape e) allant de 1 à 1000 Pa.s, mieux de 20 à 500 Pa.s, en particulier de 40 à 200 Pa.s.

*Mesure des caractéristiques viscoélastiques*

Les compositions conformes à l'invention ont avantageusement un comportement viscoélastique, à caractère élastique dominant.

De façon générale, un matériau est dit viscoélastique quand, sous l'effet du cisaillement, il possède à la fois les caractéristiques d'un matériau élastique, c'est à dire capable de stocker de l'énergie et les caractéristiques d'un matériau visqueux, c'est à dire capable de dissiper de l'énergie.

Plus particulièrement, le comportement viscoélastique des compositions conformes à l'invention peut être caractérisé par son module de rigidité G*, son élasticité $\delta$ et son seuil d'écoulement $\tau_c$ ; ces paramètres sont notamment définis dans l'ouvrage « Initiation à la rhéologie », G. Couarraze et J.L. Grossiord, 2ème édition, 1991, Edition Lavoisier-Tec 1 Doc.

Les mesures sont effectuées sur un rhéomètre à contrainte imposée, « Haake RheoStress 600® » de la société ThermoRhéo, équipé d'un bain thermostaté et d'un mobile en acier inoxydable à géométrie plan/plan, le plan ayant un diamètre de 20 mm et un entrefer (distance entre le plan inférieur - appelé plan stator - sur lequel est déposée la composition et le plan supérieur - appelé plan rotor) de 1 mm. Les 2 plans sont striés pour limiter les phénomènes de glissement aux parois des plans. Un dispositif anti-évaporation (cloche à solvants) est utilisé.

Les mesures sont effectuées à 20°C $\pm$ 1 °C.

La composition est soumise à un cisaillement harmonique selon une contrainte $\tau(t)$ variant de façon sinusoïdale selon une pulsation $\omega$ ($\omega = 2\Pi\nu$), $\nu$ étant la fréquence du cisaillement appliqué.

La contrainte $\tau(t)$ et la déformation $\gamma(t)$ sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. $\delta$ est l'angle de déphasage entre la contrainte et la déformation et G* correspond au rapport $\tau_o$ sur $\gamma_o$.

Les mesures sont effectuées à une fréquence fixe de 1 Hz ($\nu$= 1 Hz).

Dans un premier temps, l'échantillon est mis en température à 20°C $\pm$ 1 °C pendant 300 secondes.

On applique ensuite des contraintes croissantes à l'échantillon en partant d'une contrainte initiale égale à 0,01 Pa pour arriver à une contrainte finale de 1000 Pa, pour aller jusqu'à la destruction de l'échantillon (les contraintes n'étant appliquées qu'une seule fois).

On mesure l'évolution du module de rigidité G* (correspondant au rapport de $\tau_o$ sur $\gamma_o$.) et le déphasage $\delta$ (correspondant à l'angle de déphasage de la contrainte appliquée par rapport à la déformation mesurée) en fonction de la contrainte $\tau$(t) appliquée.

On mesure en particulier la déformation de la composition pour la zone de contrainte dans laquelle la variation du module de rigidité G* et de l'élasticité $\delta$ est inférieure à 7 % (zone des microdéformations) et on détermine ainsi les paramètres dits « plateaux » Gp et $\delta_p$.

La contrainte seuil $\tau_c$ (la composition ne s'écoulant pas sous son propre poids, la contrainte seuil correspondant à la force minimale qu'il est nécessaire d'appliquer à la composition pour provoquer son écoulement) est déterminée à partir de la courbe G* = f($\tau$). On la définit comme la valeur de $\tau$ à l'intersection des 2 tangentes à la courbe G*=f($\tau$ pour les faibles valeurs de $\tau$ et pour les fortes valeurs de $\tau$.

Le comportement viscoélastique des compositions selon l'invention est notamment caractérisé par un module de rigidité plateau Gp supérieur à 100 Pa, de préférence supérieur à 500 Pa.

C'est pourquoi l'invention a encore pour objet une composition de vernis à ongles comprenant un milieu cosmétiquement acceptable et au moins un agent épaississant thixotrope, ladite composition présentant un module de rigidité plateau Gp supérieur à 100 Pa.

En particulier, les compositions selon l'invention possèdent un module de rigidité plateau Gp allant de 100 à $2.10^6$ Pa.s, de préférence de $5.10^2$ à $10^4$ Pa.s, mieux de 800 à 4000 Pa.s, et en particulier de 1000 à 3000 Pa.s.

Les compositions conformes à l'invention peuvent par ailleurs présenter une élasticité $\delta_p$ allant de 2˚ à 30˚, et en particulier allant de 15˚ à 25˚ et un seuil d'écoulement $\tau_c$ allant de 10 Pa à $3.10^4$ Pa, et en particulier allant de 30 Pa à 500 Pa et mieux de 50 à 200 Pa.

### Agent épaississant

La composition selon l'invention comprend un agent épaississant thixotrope en une quantité suffisante pour conférer à la composition une viscosité au repos suffisante pour lui donner sa texture, et un comportement thixotrope. En particulier, la nature et/ou la quantité dudit agent épaississant sont telle(s) que, en réponse à une action non chimique, notamment mécanique, préalablement ou simultanément à l'application de la composition sur les ongles, la viscosité de la composition peut être abaissée de manière réversible à une valeur n'excédant pas 0,4 Pa.s, de préférence à une valeur n'excédant pas 0,3 Pa.s.

L'agent épaississant thixotrope peut être présent en une teneur supérieure ou égale à 1,7% en poids, allant par exemple de 1,7 % à 15 % en poids, par rapport au poids total de la composition, de préférence supérieure ou égale à 2% en poids, allant par exemple de 2 % à 10% en poids, préférentiellement allant de 2 % à 7,5 % en poids, préférentiellement allant de 3% à 7,5% en poids, préférentiellement allant de 3,5% à 7,5% en poids.
L'agent épaississant peut être choisi parmi les argiles hydrophiles ou organophiles, les silices pyrogénées hydrophiles ou hydrophobes, les organopolysiloxanes élastomériques et leurs mélanges.

Les argiles sont des silicates contenant un cation pouvant être choisi parmi les cations de calcium, de magnésium, d'aluminium, de sodium, de potassium, de lithium et leurs mélanges. On entend par argile hydrophile une argile apte à gonfler dans l'eau ; cette argile gonfle dans l'eau et forme après hydratation une dispersion colloïdale.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.
Ces argiles peuvent être d'origine naturelle ou synthétique.

Comme argile hydrophile, on peut citer les smectites telles que les saponites, les hectorites, les montmorillonites, les bentonites, la beidellite et en particulier les hectorites synthétiques (appelées aussi laponites) comme les produits vendus par la société Laporte sous le nom Laponite XLG, Laponite RD, Laponite RDS (ces produits sont des silicates de sodium et de magnésium et en particulier des silicates de sodium, de lithium et de magnésium) ; les bentonites comme le produit vendu sous la dénomination Bentone HC par la société RHEOX ; les silicates de magnésium et d'aluminium notamment hydratés comme les produits vendus par la société Vanderbilt Company sous le nom Veegum ultra, Veegum HS, Veegum DGT, ou encore les silicates de calcium et notamment celui sous forme synthétique vendu par la société sous le nom de Micro-cel C.
Les argiles organophiles sont des argiles modifiées par des composés chimiques rendant l'argile apte à gonfler dans les milieux solvants.

L'argile peut être choisie parmi la montmorrillonite, la bentonite, l'hectorite, l'attapulgite, la sépiolite, et leurs mélanges. L'argile est de préférence une bentonite ou une hectorite.

Les argiles organophiles sont des argiles modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Elementis, Tixogel VP par la société United catalyst, Claytone 34, Claytone 40, Claytone XL par la société Southern Clay; les stéaralkonium bentonites telles que celles vendues sous les dénominations Bentone 27V par la société Elementis, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées hydrophiles peuvent être obtenues par hydrolyse à haute température d'un composé volatil du silicium dans une flamme oxhydrique, produisant une silice finement divisée. Les silices hydrophiles présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes.

Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

Les organopolysiloxanes élastomériques sont en général partiellement ou totalement réticulés et éventuellement de structure tridimensionnelle.

Les organopolysiloxanes élastomères associés à une phase grasse se présentent généralement sous forme de gel constitué d'un organopolysiloxane élastomère associé à une phase grasse, inclus dans au moins une huile hydrocarbonée et/ou une huile siliconée. Ils peuvent être choisis notamment parmi les polymères réticulés décrits dans la demande EP-A-0295886.

Selon cette demande, les organopolysiloxanes élastomères sont obtenus par réaction d'addition et de réticulation d'au moins :

(a) un organopolysiloxane ayant au moins deux groupes alcényle inférieurs par molécule ;
(b) un organopolysiloxane ayant au moins deux atomes d'hydrogène liés à un atome de silicium par molécule ; et
(c) et un catalyseur du type platine.

Les organopolysiloxanes élastomériques associés à une phase grasse peuvent être choisis aussi parmi ceux décrits dans le brevet US-A-5266321, notamment parmi les polyorganopolysiloxanes comprenant des motifs $R_2SiO$ et $RSiO_{1,5}$ et éventuellement des motifs $R_3SiO_{0,5}$ et/ou $SiO_2$ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs $R_2SiO$ sur les motifs $RSiO_{1,5}$ varie de 1/1 à 30/1 ;

Selon un mode de réalisation préféré, l'agent épaississant thixotrope, est choisi parmi les argiles modifiées organophiles telles que l'hectorite modifiée par le stéarate de benzyl di methyl ammonium.

Selon un mode de réalisation avantageux, l'agent épaississant thixotrope comprend, outre une argile modifiée organophile, une silice pyrogénée hydrophile.

## Agent épaississant additionnel

La composition selon l'invention peut comprendre, en outre, un agent épaississant additionnel différent des agents épaississants thixotropes décrits précédemment.

Cet agent épaississant additionnel n'est pas apte à lui seul à rendre à la composition le caractère thixotrope (épaississant non thixotrope) ; il permet notamment d'ajuster la viscosité de la composition pour obtenir un écoulement homogène.

L'agent épaississant additionnel peut être choisi, selon le milieu cosmétiquement acceptable de la composition, parmi :

- les agents épaississants hydrophiles tes que
la gomme de guar, la gomme de guar quaternisée, les gommes de guar non-ioniques, les gommes de xanthane, de caroube, de scléroglucane, de gellane, de rhamsan, de karaya, les alginates, la maltodextrine, l'amidon et ses dérivés, l'acide hyaluronique et ses sels,

  - les polymères poly(métha)crylates de glycéryle
  - la polyvinylpyrrolidone,
  - l'alcool polyvinylique,
  - les polymères et les copolymères réticulés d'acrylamide,
  - les polymères associatifs et notamment les polyuréthanes associatifs,

- les agents épaississants organophiles comme :

- les gommes de guar alkylées (avec groupe alkyle en $C_1$-$C_6$), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les résines de polyamides comprenant des groupes alkyles ayant de 12 à 22 atomes de carbone, telles que celles décrites dans US-A-5783657.

- les agent épaississant hydrophobes tels que les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

L'agent épaississant additionnel peut être présent en une teneur allant de 0,1 % à 20 % en poids, par rapport au poids total de la composition, et de préférence allant de 0,1 % à 10 % en poids.

## Milieu solvant organique

La composition selon l'invention peut comprendre un milieu solvant organique comprenant au moins un solvant organique choisi parmi :

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tel que le carbonate de propylène, le carbonate de diméthyle,
- les acétals tels que méthylal ;
- et leurs mélanges.

Le milieu solvant organique peut représenter de 30 à 97 % en poids et notamment de 50 à 95 % en poids par rapport au poids total de la composition.

La composition selon l'invention peut comprendre un milieu aqueux.

La teneur en milieu aqueux de la composition peut aller de 5 à 95 % en poids par rapport au poids total de la composition, de préférence de 50 à 70% en poids.

## Polymère filmogène

La composition comprend avantageusement au moins un polymère filmogène.

Selon la présente invention, on entend par "polymère filmogène", un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société

ROHM & HAAS, la résine polyuréthane "TRIXENE PR 4127" de la société BAXENDEN.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre. De tels polymères sont décrits par exemple dans les documents EP 1411069 ou WO04/028488.

Le polymère filmogène peut être présent dans la composition selon l'invention en une teneur en matières sèches allant de 0,1 % à 60 % en poids, par rapport au poids total de la composition, de préférence allant de 2 % à 40 % en poids, et mieux de 5 % à 25 % en poids.

## Agent auxiliaire de filmification

Pour améliorer les propriétés filmogènes de la composition de vernis à ongles un agent auxiliaire de filmification peut être prévu.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :

- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates ;
- des dérivés oxyéthylénés, tels que les huiles oxyéthylénées, notamment les huiles végétales, telles que l'huile de ricin ; et
- leurs mélanges.

Le type et la quantité d'agent plastifiant et/ou de coalescence peuvent être choisis par l'homme du métier sur la base de ses connaissances générales.

Par exemple, la teneur en agent plastifiant et/ou de coalescence peut aller de 0,01 % à 20% et en particulier de 0,5 % à 10 % en poids par rapport au poids total de la composition.

## Agent d'étalement

Selon un mode de réalisation, la composition selon l'invention comprend un agent d'étalement choisi destiné à favoriser l'application de la composition sur les ongles. Il peut être choisi parmi les huiles de silicones linéaires ou cycliques, notamment celles ayant une viscosité $\leq 6$ centistokes ($6.10^{-6}$ $m^2/s$), et ayant notamment de 3 à 6 atomes de silicium, ces silicones comportant éventuellement un ou plusieurs groupes alkyles ou alkoxy ayant de 1 ou 2 atomes de carbone. Comme huile de silicone utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'agent d'étalement peut représenter de 0,1 à 15% en poids par rapport au poids total de la composition, de préférence de 0,5 à 10% en poids et mieux de 1 à 5% en poids.

**Agent de coloration**

Selon une alternative, l'agent de coloration est choisi parmi les corps magnétiques, les pigments diffractants, les pigments interférentiels, les particules réfléchissantes et leurs mélanges

L'agent de coloration peut être présent en une teneur supérieure ou égale à 2,5% en poids, de préférence supérieure ou égale à 3% en poids, mieux, supérieure ou égale à 3,5% en poids, et encore mieux, supérieure ou égale à 4% en poids, pouvant aller jusqu'à 20%, de préférence jusqu'à 15% en poids, et mieux jusqu'à 10% en poids par rapport au poids total de la composition.

A/ Particules magnétiques

Par « particules magnétiques », encore appelées corps magnétiques, on désigne des particules présentant une susceptibilité magnétique, c'est-à-dire sensible à l'action d'un champ magnétique et tendant par exemple à s'aligner sur les lignes de champ.

Les particules magnétiques peuvent comporter tout matériau magnétique présentant une sensibilité aux lignes d'un champ magnétique, qu'il soit produit par un aimant permanent ou issu d'une induction, ce matériau étant par exemple choisi parmi le nickel, le cobalt, le fer, leurs alliages et oxydes, notamment $Fe_3O_4$, et aussi le gadolinium, le terbium, le dysprosium, l'erbium, leurs alliages et oxydes. Le matériau magnétique peut être de type « doux » ou « dur ». Les particules magnétiques peuvent présenter ou non une structure multicouche, comportant au moins une couche d'un matériau magnétique, tel que par exemple le fer, le nickel, le cobalt, leurs alliages et oxydes, notamment $Fe_3O_4$. Les particules magnétiques présentent de préférence un caractère anisotrope. Les particules magnétiques sont de préférence asphériques, présentant par exemple une forme allongée. Ainsi, lorsque ces particules sont soumises à un champ magnétique, elles tendent à s'orienter avec leur axe longitudinal dans l'alignement des lignes de champ, et subissent un changement d'orientation qui se traduit par un changement d'aspect provenant de l'anisotropie et créant le ou les motifs.
Lorsque les particules magnétiques sont sensiblement sphériques, de préférence leur aspect est inhomogène, de manière à ce qu'un changement d'orientation induise un changement d'aspect.
La composition peut prendre un état empêchant tout nouveau changement d'orientation des particules magnétiques sous l'effet d'un champ magnétique après une durée de séchage donnée.

La dimension des particules magnétiques est par exemple comprise entre 1 nm et 700 $\mu$m, par exemple entre 1 $\mu$m et 500 $\mu$m, mieux encore entre environ 10 $\mu$m et environ 150 $\mu$m. Par « dimension », on désigne la dimension donnée par la distribution granulométrique statistique à la moitié de la population, dite D50.

Les particules magnétiques peuvent comporter des pigments magnétiques.
Des pigments convenant tout particulièrement sont les nacres comportant de l'oxyde de fer $Fe_3O_4$. Des pigments présentant des propriétés magnétiques sont par exemple ceux commercialisés sous les dénominations commerciales Colorona Blackstar Blue, Colorona Blackstar Green, Colorona Blackstar Gold, Colorona Blackstar Red, Microna Matte Black (17437), Mica Black (17260), Colorona Patina Silver (17289) et Colorona Patina Gold (17288) de la société MERCK, Gemtone Moonstone (G 004) ou Chroma-Lite Black (4498) de la société ENGELHARD.
A titre encore d'exemple de pigment magnétique susceptible d'entrer dans la formulation de la composition, on peut citer les particules d'oxyde de fer noir, par exemple celles commercialisées sous la dénomination SICOVIT noir E172 par la société BASF.
Les pigments magnétiques peuvent encore comporter du fer métal, notamment du fer doux passivé, par exemple obtenu à partir de fer carbonyle en mettant en oeuvre le procédé décrit dans le brevet US 6 589 331, dont le contenu est incorporé par référence. Ces particules peuvent comporter une couche d'un oxyde en surface.

B/ Pigment interférentiel

L'expression « pigment interférentiel » désigne un pigment capable de produire une couleur par un phénomène d'interférences, par exemple entre la lumière réfléchie par une pluralité de couches superposées d'indices de réfraction différents, notamment une succession de couches de haut et de bas indices de réfraction.
Un pigment interférentiel peut par exemple comporter plus de quatre couches d'indices de réfraction différents.
Les couches du pigment interférentiel peuvent entourer ou non un noyau, lequel peut présenter une forme aplatie ou non.
Les nacres sont des exemples de pigments interférentiels.

Nacres

Par « nacre », il faut comprendre des particules colorées de toute forme, irisées ou non, notamment produites par certains mollusques dans leur coquille ou bien synthétisées et qui présentent un effet de couleur par interférence optique.

Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth.

Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être introduites en tant que pigment interférentiel dans la première composition, on peut citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

## Particules réfléchissantes interférentielles

Ces particules peuvent être choisies parmi les particules à substrat synthétique enrobé au moins partiellement d'au moins une couche d'au moins un oxyde métallique, choisi par exemple parmi les oxydes de titane, notamment $TiO_2$, de fer notamment $Fe_2O_3$, d'étain, de chrome, le sulfate de baryum et les matériaux suivants : $MgF_2$, $CrF_3$, ZnS, ZnSe, $SiO_2$, $Al_2O$, MgO, $Y_2O_3$, $SeO_3$, SiO, $HfO_2$, $ZrO_2$, $CeO_2$, $Nb_2O_5$, $Ta_2O_5$, $MoS_2$ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD. Des particules à substrat de verre revêtu d'oxyde métallique, notamment de Ti02, sont commercialisées par la société Nippon Sheet Glass sous la dénomination Methashine.

## Pigment goniochromatique

Par « pigment goniochromatique », on désigne au sens de la présente invention un pigment permettant d'obtenir, lorsque la composition est étalée sur un support, un trajet de couleur dans le plan a*b* de l'espace colorimétrique CIE 1976 qui correspond à une variation Dh˚ de l'angle de teinte h˚ d'au moins 20˚ lorsque l'on fait varier l'angle d'observation par rapport à la normale entre 0˚ et 80˚, pour un angle d'incidence de la lumière de 45˚.

Le trajet de couleur peut être mesuré par exemple au moyen d'un spectrogonioréflectomètre de marque INSTRUMENT SYSTEMS et de référence GON 360 GONIOMETER, après que la première composition a été étalée à l'état fluide avec une épaisseur de 300 μm au moyen d'un étaleur automatique sur une carte de contraste de marque ERICHSEN et de référence Typ 24/5, la mesure étant effectuée sur le fond noir de la carte.

Le pigment goniochromatique peut être choisi par exemple parmi les structures multicouche interférentielles et les agents de coloration à cristaux liquides.

Dans le cas d'une structure multicouche, celle-ci peut comporter par exemple au moins deux couches, chaque couche étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O$, Pt, Va, $Al_2O$, MgO, $Y_2O_3$, $S_2O$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

La structure multicouche peut présenter ou non, par rapport à une couche centrale, une symétrie au niveau de la nature chimique des couches empilées. Selon l'épaisseur et la nature des différentes couches, on obtient différents effets.

Des exemples de structures multicouche interférentielles symétriques sont par exemple les structures suivantes : $Fe_2O_3/SiO_2/Fe_2O/SiO_2/Fe_2O_3$, un pigment ayant cette structure étant commercialisé sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$; $TiO_2/SiO_2/TiO_2$ et $TiO_2/Al_2O_3/TiO_2$, des pigments ayant ces structures étant commercialisés sous la dénomination XIRONA par la société MERCK (Darmstadt).

Les agents de coloration à cristaux liquides comprennent par exemple des silicones ou des éthers de cellulose sur lesquels sont greffés des groupes mésomorphes. Comme particules goniochromatiques à cristaux liquides, on peut utiliser par exemple celles vendues par la société CHENIX ainsi que celles commercialisées sous la dénomination HELICONE® HC par la société WACKER.

Comme pigment goniochromatique, on peut encore utiliser certaines nacres, des pigments à effets sur substrat synthétique, notamment substrat type alumine, silice, borosilicate, oxyde de fer, aluminium, ou des paillettes interférentielles issues d'un film de polytéréphthalate.

Le matériau peut en outre comporter des fibres goniochromatiques dispersées. De telles fibres pourront présenter une longueur inférieure à 80 $\mu$m par exemple.

C/ Pigment diffractant

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière. Un tel pigment est encore parfois appelé pigment holographique.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article *« Pigments Exhibiting Diffractive Effects »* d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinusoïdaux, en escalier.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non, de même linéature ou non.

Le pigment diffractant peut présenter une structure multicouche comportant une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : $MgF_2$, $SiO_2$, $Al_2O$, $AlF_3$, $CeF_3$, $LaF_3$, $NdF_3$, $SmF_2$, $BaF_2$, $CaF_2$, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr, Fe et leurs matériaux, associations, alliages et leur dopage par des terres rares.

Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : $MgF_2$, SiO, $SiO_2$, $Al_2O$, $TiO_2$, WO, AIN, BN, $B_4C$, WC, TiC, TiN, $N_4Si_3$, ZnS, des particules de verre, des carbones de type diamant et leurs associations.

En variante, le pigment diffractant peut être composé d'un matériau diélectrique ou céramique préformé tel qu'un minéral en lamelle naturelle, par exemple du mica peroskovite ou du talc, ou des lamelles synthétiques formées à partir de verre, d'alumine, de $SiO_2$, de carbone, d'un oxyde de fer/mica, de mica recouvert de BN, de BC, de graphite, d'oxychlorure de bismuth, et leurs associations.

A la place d'une couche d'un matériau diélectrique, d'autres matériaux améliorant les propriétés mécaniques peuvent convenir. De tels matériaux peuvent comporter du silicone, des silicides métalliques, des matériaux semi-conducteurs formés à partir d'éléments des groupes III, IV et V, des métaux ayant une structure cristalline cubique centrée, des compositions ou matériaux de cermet, des verres semi-conducteurs, et leurs associations variées.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante : $MgF_2/Al/MgF_2$, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du $MgF_2$ peut être comprise entre 80 et 95 % du poids total du pigment.

D'autres pigments diffractants sont commercialisés sous les dénominations Metalure® Prismatic par la société ECKART®.

D'autres structures possibles sont Fe/Al/Fe ou Al/Fe/Al.

La dimension du pigment diffractant peut être comprise par exemple entre 5 et 200 $\mu$m, mieux entre 5 et 100 $\mu$m, par exemple entre 5 et 30 $\mu$m.

L'épaisseur des particules de pigment diffractant peut être inférieure ou égale à 3 $\mu$m, mieux 2 $\mu$m, par exemple de l'ordre de 1 $\mu$m.

D/ Pigments ou particules réfléchissantes

Par « particules réfléchissantes », on désigne des particules dont la taille, la structure, notamment l'épaisseur de la ou des couches qui la constituent et leurs natures physique et chimique, et l'état de surface, leur permettent de réfléchir la lumière incidente. Cette réflexion peut, le cas échéant, posséder une intensité suffisante pour créer à la surface de la composition ou du mélange, lorsque celui-ci est appliqué sur le support à maquiller, des points de surbrillance visibles à l'oeil nu, c'est-à-dire des points plus lumineux qui contrastent avec leur environnement en semblant briller.

Les particules réfléchissantes peuvent être sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Ces particules peuvent présenter des formes variées, notamment être en forme de plaquettes ou globulaires, en particulier sphériques.

Les particules réfléchissantes, quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, notamment des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou matériau métallique. Le substrat peut être monomatière, multimatériau, organique et/ou inorganique.

Plus particulièrement, il peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative. Le matériau réfléchissant peut comporter une couche de métal ou d'un matériau métallique.

Des particules réfléchissantes sont décrites notamment dans les documents JP-A-09188830, JP-A-1 0158450, JP-A-1 0158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent.

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

On peut également utiliser des particules comprenant un substrat métallique tel que l'argent, l'aluminium, le fer, le chrome, le nickel, le molybdène, l'or, le cuivre, le zinc, l'étain, le magnésium, l'acier, le bronze, le titane, ledit substrat étant enrobé d'au moins une couche d'au moins un oxyde métallique tels que l'oxyde de titane, l'oxyde d'aluminium, l'oxyde de fer, l'oxyde de cérium, l'oxyde de chrome, les oxydes de silicium et leurs mélanges.

On peut citer à titre d'exemple les poudres d'aluminium, de bronze ou de cuivre enrobées de SiO2 commercialisées sous la dénomination VISIONAIRE par la société ECKART.

**Matière colorante additionnelle**

Selon l'alternative décrite ci-dessus, la composition selon l'invention peut comprendre, outre les agents de coloration choisis parmi les corps magnétiques, les pigments diffractants, les pigments interférentiels, les particules réféchissantes, au moins un agent de coloration dit additionnel, notamment qui produisant une couleur par absorption d'au moins une partie du spectre visible.

Ces matières colorantes additionnelles peuvent être choisies parmi les colorants hydrosolubles ou liposoblubles, les pigments organiques ou inorganiques ou hybrides.

L'agent de coloration peut être un composé particulaire ou non.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

L'agent de coloration peut encore être une laque ou un pigment organique choisi parmi les matériaux ci-dessous et leurs mélanges :

- le carmin de cochenille,
- les pigments organiques de colorants azoïques, anthraquinoniques, indigoïdes, xanthéniques, pyréniques, quinoliniques, de triphénylméthane, de fluorane, en particulier les pigments de type D & C,
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Les matériaux chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

Les agents de coloration additionnels peuvent être présents en une teneur allant de 0,01% à 30 % en poids, par rapport au poids de la composition, de préférence de 1 % à 10 % en poids.

Selon une autre alternative, la composition selon l'invention peut comprendre une ou des matières colorantes choisies parmi les agents de coloration et la matière colorante additionnelle décrits ci-dessus. Dans ce cas, les matières colorantes peuvent être présentes, dans la composition, en une teneur allant de 0,01 % à 60 % en poids, par rapport au poids de la composition, de préférence de 1 % à 40 % en poids.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

**Autres Additifs**

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

**Applicateur**

L'applicateur utilisé dans la présente invention comporte une lame souple définissant une face applicatrice.

De préférence, la lame souple est incurvée de manière à définir une face applicatrice généralement concave et peut présenter un bord distal convexe, lorsqu'observée de dessus.

Selon un mode de réalisation l'applicateur comporte une lame souple incurvée ayant une face applicatrice définissant une surface développable, par exemple cylindrique.

Selon un autre mode de réalisation l'applicateur comporte une lame souple incurvée définissant une face applicatrice généralement concave et ayant un bord distal convexe lorsqu'observée de dessus.

Selon un mode de réalisation, le récipient contenant le vernis à ongles est configuré pour permettre le prélèvement du produit sans passer au travers d'un organe d'essorage.

Les caractéristiques présentées ci-dessus s'appliquent à l'applicateur considéré isolément ainsi qu'à l'ensemble comportant l'applicateur et le récipient.

L'invention peut permettre à l'utilisateur de doser relativement précisément la quantité de produit prélevée par l'applicateur, compte tenu de la consistance du produit et/ou de la manière dont le produit est prélevé.

La forme incurvée de la lame souple peut notamment faciliter le prélèvement du produit.

De plus, l'absence de faisceau de poils permet d'éviter la formation de stries lors du dépôt du produit sur l'ongle.

Enfin, la forme de la lame peut faciliter l'application sur les ongles et peut permettre de réaliser rapidement un maquillage de qualité.

Dans un exemple de mise en oeuvre de l'invention, la lame souple présente une face applicatrice entièrement lisse.

La lame souple peut comporter des reliefs sur les côtés longitudinaux de la face applicatrice, par exemple des nervures.

Ces reliefs peuvent, en prenant appui sur le doigt et/ou sur les côtés de l'ongle, permettre d'écarter une portion au moins de la face applicatrice d'une distance prédéfinie de l'ongle, ce qui peut faciliter la formation d'un dépôt de produit d'épaisseur contrôlée.

Dans un exemple de mise en oeuvre de l'invention, la lame souple définit des surfaces d'appui opposées pour les doigts, de manière à permettre à l'utilisateur de modifier comme il le souhaite la courbure de la face applicatrice en serrant plus ou moins la lame souple entre ses doigts. Ces surfaces d'appui peuvent être définies, par exemple, par des ailes s'étendant de part et d'autre d'une paroi supérieure de la lame, qui en définit le dos.

La lame souple peut présenter une épaisseur non constante, afin par exemple de permettre une flexibilité accrue lors de l'utilisation. Cela peut permettre de réduire la pression exercée par la lame sur l'ongle et d'améliorer le confort du maquillage et la qualité de celui-ci.

Lorsque la lame souple comporte une paroi supérieure en définissant le dos avec des ailes disposées de part et d'autre de cette paroi supérieure, cette dernière peut présenter une épaisseur de matière plus petite qu'au niveau des ailes. Lors de l'application, la paroi supérieure peut s'incurver plus facilement. Dans une variante, les ailes présentent une paroi plus épaisse que le dos. Cela peut permettre de déformer plus facilement le bord distal de la lame en appuyant sur les ailes, en transmettant mieux l'effet exercé. L'utilisateur peut maîtriser plus facilement la déformation du bord distal. Cela peut encore permettre d'éloigner davantage la zone d'appui des doigts de celle portant le produit.

La lame souple peut se raccorder à un manche, par exemple par l'intermédiaire d'une portion plus épaisse qui peut être pleine et de section transversale circulaire ou autre. La lame souple peut être réalisée d'une seule pièce avec le manche.

La lame souple peut être réalisée dans un thermoplastique élastomère ou dans toute autre matière pouvant conférer à la lame une certaine flexibilité et compatible avec le ou les solvants éventuels contenus dans le produit.

La lame souple peut être réalisée dans une matière élastiquement déformable présentant une dureté 25 à 60 Shore A, par exemple.

La lame souple peut être réalisée éventuellement dans un métal souple ou dans un complexe comportant plusieurs couches de matériaux différents, laminées ensemble ou réunies par surinjection.

La souplesse de la lame peut, dans un exemple de mise en oeuvre de l'invention, être telle que la lame s'aplatisse sensiblement lorsque pressée contre une surface plane.

La lame peut comporter dans certaines parties du moins une armature destinée à la renforcer et/ou une charge de fibres. Comme mentionné plus haut, la lame souple présente avantageusement un bord distal convexe, lorsqu'observée de dessus. Le bord distal présente par exemple, à son sommet, un rayon de courbure compris entre 4 et 20 mm.

La lame souple peut présenter une forme générale symétrique par rapport à un plan médian longitudinal.

La lame souple peut être floquée, le cas échéant.

L'applicateur peut comporter un manche sur lequel est fixée la lame. En variante, la lame souple peut être réalisée avec une partie de préhension venue de moulage et/ou de découpage avec elle. L'axe longitudinal de la lame peut être confondu avec l'axe longitudinal du manche ou de la partie de préhension ou faire un angle non nul avec celui-ci.

Lorsqu'un manche est utilisé, la lame souple peut être fixée de manière immobile sur le manche, de façon amovible ou non. L'applicateur peut comporter, le cas échéant, un organe de réglage de la courbure de la face applicatrice, qui peut ou non être configuré pour servir d'organe de préhension.

Ainsi, la lame souple peut être fixée de façon réglable sur le manche précité. Le manche et la lame souple peuvent coopérer de manière à permettre à l'utilisateur de faire varier la forme de la face applicatrice, afin par exemple de l'adapter à la forme de l'ongle traité et/ou à la consistance du produit.

Lorsque l'applicateur comporte un manche et que la lame est mobile relativement au manche, celui-ci peut être agencé de manière à contraindre la lame plus ou moins selon sa position sur le manche.

L'un au moins de la lame et du manche peut comporter des reliefs permettant un réglage par incréments de la courbure de la face applicatrice. En variante, le réglage peut s'effectuer de manière continue.

Le manche peut comporter, éventuellement, un organe de réglage mobile dont le déplacement s'accompagne d'une modification de la courbure.

La modification de la courbure de la face applicatrice peut résulter d'une contrainte exercée latéralement sur deux côtés extérieurs opposés de la lame, par exemple, et /ou d'un étirement plus ou moins important de celle-ci.

La contrainte peut être exercée sur la lame par le manche ou par un organe de réglage autre, par exemple un anneau engagé sur la lame et mobile axialement relativement à celle-ci. En déplaçant l'anneau sur la lame, celle-ci peut être plus ou moins serrée et s'incurver de manière correspondante.

La lame peut être ajourée. Cela peut accroître la visibilité de l'ongle en cours de maquillage et/ou permettre une possibilité de montage de la lame sur un manche de manière à modifier assez simplement la courbure de la face applicatrice. La lame peut être ajourée, comportant une lame entre deux bords opposés de l'ouverture.

Le manche peut être agencé pour permettre d'étirer plus ou moins la lame entre deux bords opposés de l'ouverture.

La lame peut être réalisée, le cas échéant, dans une matière transparente, ce qui peut améliorer la visibilité de l'ongle pendant l'utilisation.

Avantageusement, la lame est réalisée dans une matière qui facilite son nettoyage, par exemple un polymère sur lequel le produit adhère peu, par exemple un polymère siliconé.

L'applicateur peut comporter deux faces applicatrices de dimensions différentes, ces faces applicatrices étant par exemple définies par deux lames souples disposées bout à bout. L'utilisateur peut alors choisir la lame qu'il souhaite utiliser pour l'application et se servir de l'autre lame comme d'une partie de préhension. Les deux lames peuvent être réalisées d'une seule pièce par moulage de matière ou découpe d'une feuille, suivie d'une mise en forme éventuelle.

L'applicateur peut être à usage unique ou non.

L'ensemble peut être proposé à l'utilisateur avec plusieurs applicateurs correspondant par exemple au nombre d'utilisations attendu, compte-tenu de la quantité de produit contenue dans le récipient.

Les applicateurs peuvent être séparés les uns des autres ou proposés à l'utilisation avec au moins deux applicateurs solidaires d'un même support. Cela peut faciliter, le cas échéant, la fabrication et le conditionnement, les applicateurs étant par exemple moulés et/ou prédécoupés avec le support et séparés du support par l'utilisateur au fur et à mesure des utilisations.

Plusieurs applicateurs peuvent par exemple être prédécoupés sur une feuille proposée à l'utilisateur avec un récipient contenant le produit, par exemple dans un emballage commun.

Lorsque plusieurs applicateurs sont solidaires d'un même support, les applicateurs peuvent être identiques et correspondre par exemple à plusieurs utilisations successives ou être différents, étant destinés par exemple respectivement aux ongles des pieds et à ceux des mains.

Le récipient peut comporter un couvercle définissant un logement pour recevoir au moins partiellement au moins un applicateur en l'absence d'utilisation.

Ce logement peut s'étendre, éventuellement, selon un axe oblique relativement à l'axe du récipient, afin d'accroître la stabilité de l'ensemble.

L'invention a encore pour objet, selon un autre de ses aspects, un procédé de maquillage des ongles, comportant les étapes suivantes :

- prélever le produit dans un récipient en utilisant la lame d'un applicateur tel que défini plus haut,
- appliquer le produit en utilisant la face applicatrice de la lame.

Dans un tel procédé, avant ou après le prélèvement du produit, l'utilisateur peut adapter la courbure de lame à celle de l'ongle, soit en exerçant directement une action sur la lame, soit en agissant sur un organe de réglage de la courbure.

Selon un mode de réalisation, l'applicateur comporte une lame souple définissant une face applicatrice concave, la lame étant pourvue sur ses côtés longitudinaux de reliefs destinés à venir en appui sur l'ongle et/ou le doigt au cours de l'application, afin de permettre à la face applicatrice de ménager avec l'ongle un interstice d'épaisseur prédéfinie. Les reliefs sont par exemple des nervures s'étendant sur les côtés longitudinaux de la face applicatrice. Les reliefs peuvent contribuer à contenir le produit sur la face applicatrice, le cas échéant.

Selon une variante, l'applicateur comporte une lame souple qui présente une paroi supérieure et deux ailes disposées de part et d'autre de cette paroi supérieure, la paroi supérieure présentant une épaisseur de matière différente de celle des ailes, notamment inférieure.

L'applicateur peut comporter une lame souple et un manche sur lequel est fixée la lame d'une manière réglable, la lame et le manche coopérant de manière à permettre en fonction de la position de la lame sur le manche de modifier la courbure de la face applicatrice.

Selon un mode de réalisation, l'applicateur peut comporter une lame souple définissant une face applicatrice et un organe de réglage de la courbure de la face applicatrice.

La lame souple peut être réalisée d'une seule pièce avec une partie de préhension. La lame souple est par exemple réalisée par découpage d'un matériau en feuilles ou par moulage de matière avec la partie de préhension. La partie de préhension peut éventuellement définir une face applicatrice.

La lame souple peut être ajourée, la face applicatrice s'étendant au moins partiellement de part et d'autre de cet ajour.

Le récipient peut comporter un couvercle de fermeture ou un support logé dans le récipient, pourvu d'un logement dans lequel peut être reçue au moins partiellement la lame souple en l'absence d'utilisation.

Ce logement est par exemple défini par une cheminée dans laquelle peut être insérée la lame souple en l'absence d'utilisation, la lame souple étant par exemple solidaire d'un manche qui s'étend hors du logement. Le logement peut encore être défini par l'intérieur d'une capsule de fermeture qui est elle-même munie d'un couvercle. Le support peut être agencé pour prendre appui sur l'extrémité supérieure du récipient. L'organe de fermeture peut appuyer sur le support à sa périphérie, afin de contribuer à l'étanchéité de la fermeture, par exemple.

L'invention pourra être mieux comprise à la lecture de la description détaillée qui va suivre, d'exemples de mise en oeuvre non limitatifs de celle-ci, et à l'examen du dessin annexé, sur lequel :

- la figure 1 est une coupe longitudinale, schématique, d'un exemple d'ensemble de conditionnement et d'application selon l'invention,
- la figure 2 représente isolément et partiellement l'applicateur, observé de côté,
- la figure 3 représente isolément et partiellement l'applicateur, observé de dessus,
- la figure 4 est une section transversale selon IV-IV de la figure 3,
- la figure 5 illustre le prélèvement du produit avec l'applicateur,
- la figure 6 illustre l'application sur l'ongle,
- la figure 7 illustre la possibilité qu'a l'utilisateur de modifier la courbure de la face applicatrice,
- la figure 8 est une vue analogue à la figure 4 d'une variante de réalisation,
- la figure 9 est une section transversale de l'applicateur de la figure 8 au cours de l'application du produit sur l'ongle,
- la figure 10 est une vue analogue à la figure 4 d'une variante de réalisation,
- les figures 11 à 14 représentent, de manière schématique, en vue de dessus, d'autres exemples d'applicateurs,
- les figures 15 et 16 représentent, en perspective, de manière schématique, d'autres exemples d'applicateurs,
- les figures 17 à 20 représentent d'autres exemples d'ensembles de conditionnement et d'application selon l'invention,
- la figure 21 représente un autre exemple d'applicateur,
- la figure 22 représente, de manière schématique, en perspective, une variante de réalisation de l'applicateur,
- la figure 23 est une vue de face de l'applicateur de la figure 22,
- la figure 24 est une vue de côté de l'applicateur de la figure 22,
- la figure 25 est une coupe longitudinale selon XXV-XXV de la figure 23,
- la figure 26 est une vue axiale de l'applicateur de la figure 22 depuis l'extrémité de la lame souple,
- la figure 27 représente, en vue de dessus, une variante de réalisation de l'applicateur,
- la figure 28 est une vue de dessous de l'applicateur de la figure 27,
- la figure 29 représente l'applicateur de la figure 27, observé de côté,
- la figure 30 est une coupe selon XXX-XXX de la figure 29,
- la figure 31 est une vue, en perspective, de l'applicateur de la figure 27, et
- la figure 32 représente partiellement le bord libre de la lame souple d'une variante de réalisation d'applicateur.

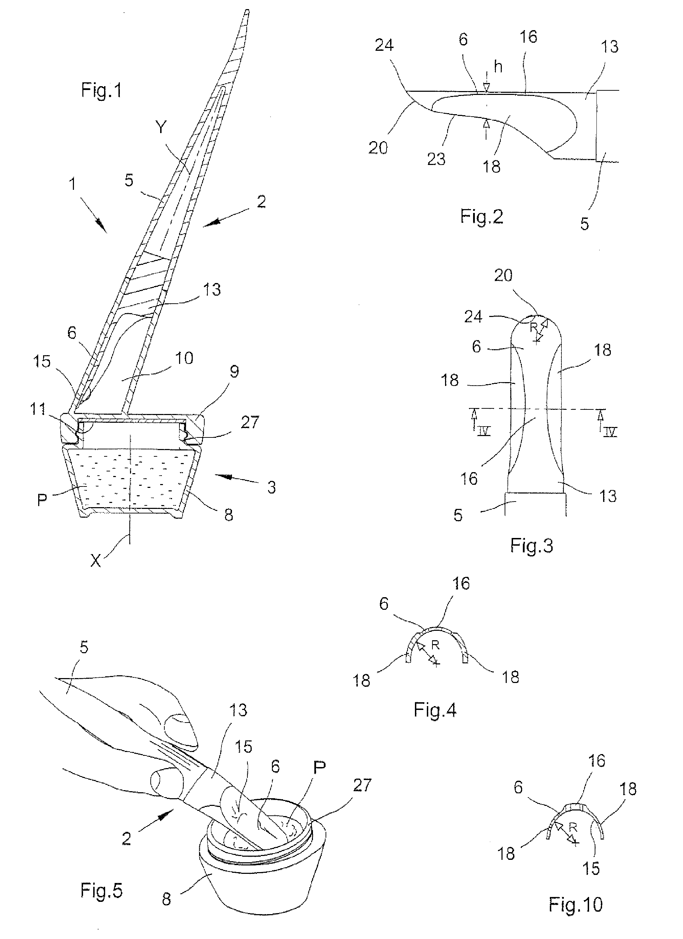

L'ensemble 1 représenté à la figure 1 comporte un applicateur 2 et un récipient 3 contenant un produit de vernis à ongles, présentant par exemple une viscosité supérieure à 0,6 Pa.S et/ou un comportement thixotrope et/ou un caractère rhéolfluidifiant.

Dans l'exemple considéré, l'applicateur 2 comporte un manche 5 et une lame souple 6 servant à l'application du produit P sur les ongles.

Le récipient 3 comporte un corps formant réservoir 8 et un couvercle 9 présentant une cheminée définissant un logement 10 pour recevoir la lame 6 en configuration de stockage, comme représenté à la figure 1.

Le couvercle 9 peut comporter un organe d'étanchéité 11 tel qu'un joint, une jupe ou un opercule, afin d'obtenir une fermeture étanche du récipient 3 en l'absence d'utilisation.

Le logement 10 peut présenter un axe longitudinal Y qui fait un angle avec l'axe X du corps de réservoir 8 de manière à améliorer la stabilité de l'ensemble 1.

Dans l'exemple considéré, la lame 6 se raccorde au manche 5 qui est creux par l'intermédiaire d'une portion d'extrémité plus épaisse 13, pleine et de section transversale circulaire. La portion d'extrémité 13 peut être réalisée de manière à obturer sensiblement l'ouverture du logement 10 lorsque l'applicateur 2 est en place. La portion d'extrémité 13 peut être réalisée dans la même matière que la lame souple 6.

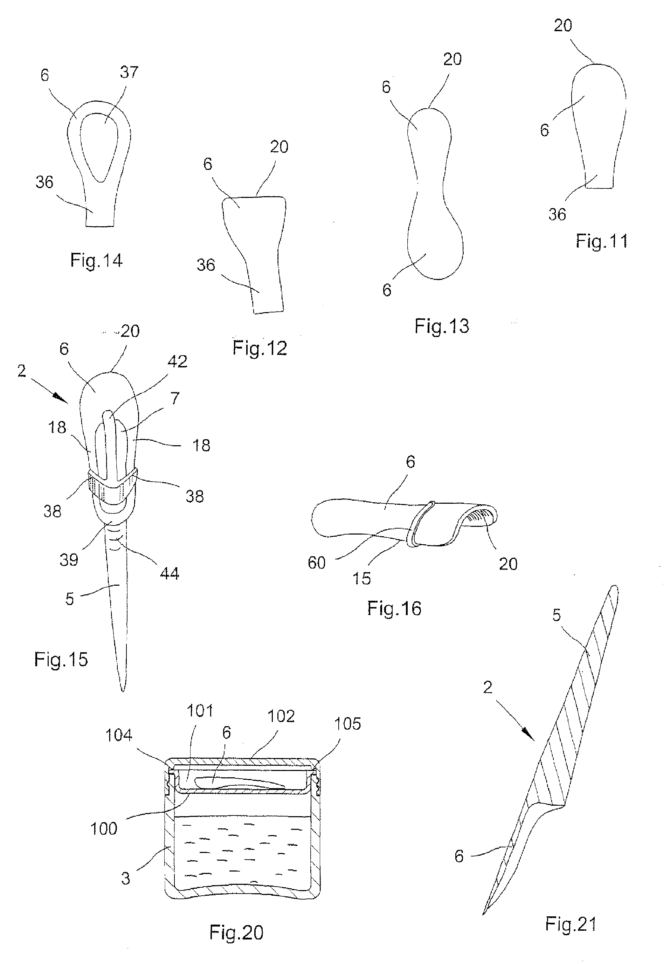

Dans une variante illustrée à la figure 20, le manche 5 est réalisé d'une seule pièce par moulage de matière avec la lame 6.

La lame 6 comporte, dans l'exemple considéré, une paroi supérieure 16 définissant le dos de la lame et deux ailes 18 se raccordant à cette paroi 16, cette dernière et les ailes 18 définissant ensemble une face applicatrice 15 de forme généralement concave. La face applicatrice 15 peut définir, dans des exemples de mise en oeuvre de l'invention, une surface développable.

Comme on peut le voir sur la figure 3, dans l'exemple considéré le bord distal 20 de la lame 6 est arrondi en vue de dessus, ayant en son sommet 24 un rayon de courbure r qui est par exemple compris entre 4 et 20 mm. La lame 6 peut être symétrique par rapport à un plan médian longitudinal.

La hauteur h des ailes 18 peut augmenter progressivement depuis le sommet du bord distal 20 vers le manche 5, comme on peut le voir sur la figure 2, passant par exemple par un point d'inflexion 23 qui peut être plus proche du sommet 24 que du manche 5.

La paroi supérieure 16 peut être réalisée, comme on peut le voir sur la figure 4, avec une épaisseur de matière réduite, de façon à présenter une flexibilité accrue.

Dans une région médiane de la lame située sensiblement à mi-distance entre le sommet 24 du bord distal 20 et le manche 5, la face applicatrice 15 peut présenter, comme illustré à la figure 4, une forme incurvée, de rayon courbure sensiblement constant R, par exemple compris entre 4 et 20 mm, par exemple de l'ordre de 7 mm.

Le cas échéant, les rayons r et/ou R peuvent être infinis, la lame ayant par exemple un bord distal rectiligne et perpendiculaire à son axe longitudinal et/ou étant plane.

Le dos de la lame peut être sensiblement rectiligne et parallèle à l'axe longitudinal du manche 5, jusqu'à la portion épaisse 13. La face applicatrice 15 peut être sensiblement cylindrique, de génératrice parallèle à l'axe du manche.

La lame 6 peut être réalisée par moulage d'une matière thermoplastique, notamment d'un élastomère thermoplastique, la forme incurvée venant de moulage. La lame 6 peut éventuellement être renforcée au moins par endroits avec une armature ou une charge de fibres.

L'ensemble 1 peut s'utiliser de la manière suivante.

L'utilisateur ouvre le récipient 3 et prélève au moyen de l'applicateur 2 le produit P à travers l'ouverture du corps 8. De préférence, ce dernier est réalisé avec un col 27 relativement large, de façon à faciliter cette opération. Le diamètre du col 27 est par exemple supérieur ou égal à 2 cm.

Pour le prélèvement, l'utilisateur peut tapoter le produit avec la lame 6, la concavité de celle-ci étant dirigée vers le produit P. En variante, l'utilisateur peut se servir de la lame 6 comme d'une cuillère, sa concavité étant orientée généralement vers le haut. Le cas échéant, l'utilisateur peut essuyer l'excès de produit prélevé sur le bord du col 27.

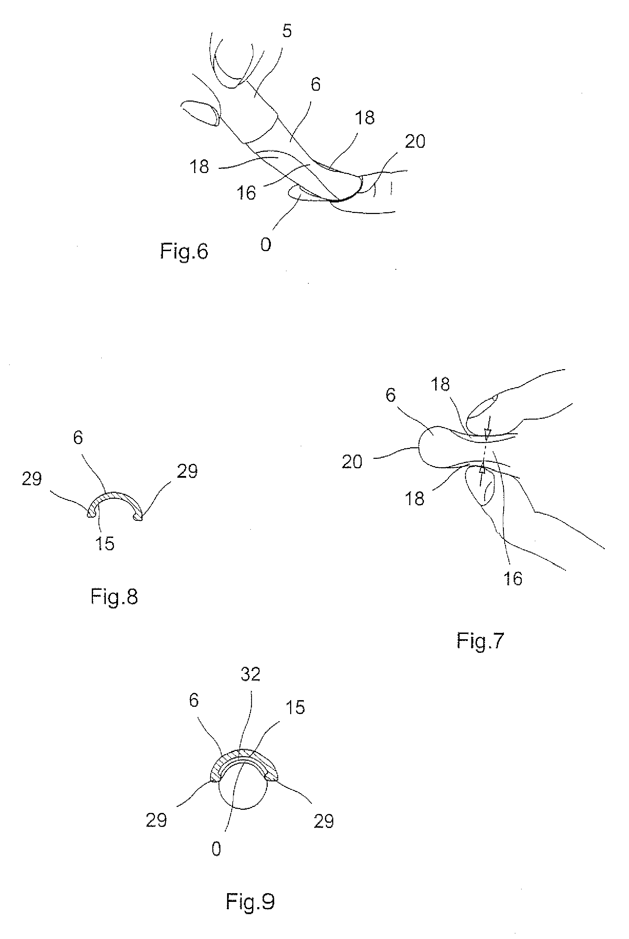

Ensuite, l'utilisateur peut amener la face applicatrice 15 au contact de l'ongle O, comme illustré à la figure 6, et appliquer le produit en déplaçant le bord distal 20 depuis la lunule en direction du bord libre de l'ongle.

Lors de l'application, la paroi supérieure 16 peut éventuellement s'incurver, devenant concave du côté opposé à la face applicatrice 15, et les ailes 18 peuvent se déformer pour accompagner la déformation de la paroi supérieure 16.

Le cas échéant, comme illustré à la figure 7, l'utilisateur peut appuyer avec ses doigts sur les ailes 18 de façon à modifier la courbure de la face applicatrice 15 et/ou du bord distal 20 afin notamment d'adapter la forme de la face applicatrice 15 et/ou celle du bord distal 20 à l'ongle traité.

L'invention n'est pas limitée à l'exemple qui vient d'être décrit.

La lame 6 peut comporter, sur ses côtés longitudinaux 8, des nervures 29 formant saillie sur la face applicatrice 15, comme illustré sur la figure 8.

Ces nervures 29 peuvent s'étendre parallèlement à l'axe longitudinal de la lame 6.

La présence des nervures 29 peut permettre de ménager entre la face applicatrice 15 et l'ongle O, comme illustré à la figure 9, un interstice 32 d'épaisseur sensiblement uniforme, afin d'obtenir plus facilement un dépôt régulier de produit.

Dans la variante illustrée à la figure 10, la paroi supérieure 16 est plus épaisse que les ailes 18.

La lame 6 peut être réalisée avec des formes très diverses et l'applicateur 2 peut comporter ou non un manche sur lequel est rapporté la lame.

La lame 6 peut par exemple être réalisée initialement sans incurvation, celle-ci pouvant provenir le cas échéant de son montage sur un manche adapté ou de sa manipulation par l'utilisateur lors de l'application.

Dans les exemples des figures 11 à 14, l'applicateur ne comporte pas de manche fabriqué séparément et la lame 6 est réalisée d'une seule pièce par moulage de matière avec une partie de préhension 36. La lame 6 peut être réalisée de façon à être plane ou incurvée au repos.

On peut réaliser le bord distal 20 avec une forme plus ou moins arrondie, voire rectiligne, en fonction par exemple de la taille de l'ongle à maquiller, comme illustré sur les figures 11 et 12. La lame 6 peut notamment présenter sur au moins une portion de sa longueur, entre ses côtés longitudinaux, une largeur qui croît en rapprochement du bord distal 20.

Le cas échéant, l'applicateur 2 peut comporter une lame 6 à chacune de ses extrémités.

Dans l'exemple de la figure 13, les deux lames 6 sont réalisées d'une seule pièce, par exemple par moulage de matière, avec des formes différentes de façon à permettre le traitement d'une plus grande variété d'ongles.

Lors de l'utilisation, l'une des lames 6 sert de partie de préhension pour l'utilisateur.

La lame 6 peut comporter un ajour 37, comme illustré à la figure 14, ce qui peut permettre d'accroître la visibilité de l'ongle lors du maquillage et peut permettre également de diminuer la quantité de produit prélevée par l'applicateur.

Lorsque la lame 6 est solidaire d'un manche, le montage de la lame 6 sur le manche peut s'effectuer de manière à permettre un réglage de la courbure de la face applicatrice 15.

Dans l'exemple de la figure 15, le manche 5 comporte deux pattes 38 qui forcent la lame 6 à s'incurver. Cette dernière peut être similaire à celle de l'exemple de la figure 14, comportant un ajour 37, dans lequel peut s'engager le manche 5, les pattes 38 venant par exemple s'appliquer du côté extérieur des ailes 18.

La lame 6 peut venir en appui contre le manche 5 par une portion proximale 39 opposée au bord distal 20.

Le manche 5 peut comporter un doigt 42 pourvu d'une encoche dans laquelle peut s'engager le bord de l'ouverture de la lame 6.

Le manche 5 peut comporter des crans 44 qui permettent d'étirer plus ou moins la lame 2 sur le manche 5, ces crans 44 étant par exemple des bossages entre lesquels peut s'engager la portion proximale 39 de la lame 6. La lame 6 est tendue entre l'encoche du doigt 42 et le cran 44 sélectionné.

Dans l'exemple de la figure 16, un organe de réglage 60 se présentant sous la forme d'un anneau engagé sur la lame 6, permet en étant déplacé sur celle-ci, d'incurver plus ou moins la face applicatrice 15.

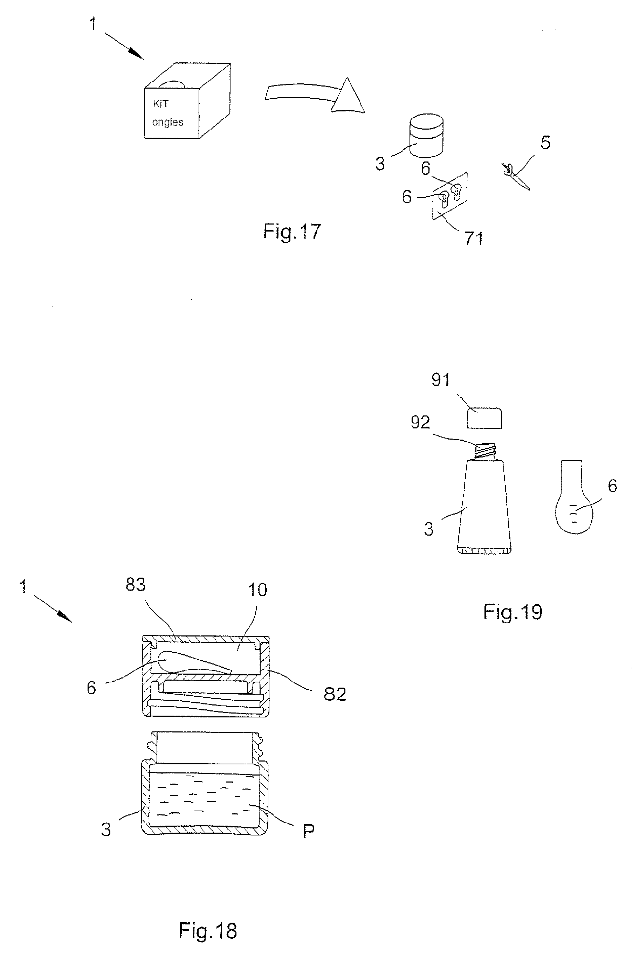

Une pluralité de lames 6 peuvent être proposées à l'utilisateur, étant par exemple prédécoupées dans une feuille 71 d'un matériau souple, comme illustré à la figure 17.

L'utilisateur peut détacher une lame 6 et la monter sur le manche 5 avant de procéder au prélèvement du produit dans le récipient.

L'ensemble 1 peut être proposé avec plusieurs lames de formes différentes ou identiques, éventuellement à usage unique, dans un même emballage.

L'ensemble 1 représenté à la figure 18 comporte un récipient 3 qui se présente sous la forme d'un pot à large col.

L'applicateur 2 peut être logé dans un logement 10 d'une capsule de fermeture 82 du récipient. Ce logement 10 peut être fermé par un couvercle 83.

Dans l'exemple de la figure 19, le produit est contenu dans un récipient 3 qui se présente sous la forme d'un tube souple pourvu d'un bouchon 91. Pour prélever le produit, l'utilisateur appuie sur le tube et place la sortie du col 92 du tube au-dessus de la face applicatrice, pour y déposer le produit.

Dans l'exemple de la figure 20, la lame 6 est contenue dans un logement 101 d'un support 100 disposé à l'intérieur du récipient 3.

Un couvercle 102 vient fermer le récipient 3 et le logement 101. Le support 100 peut comporter un rebord annulaire 105 venant en appui sur l'extrémité supérieure du récipient 3. Le couvercle peut comporter un jonc 104 venant appuyer de manière étanche sur le rebord 105.

De nombreuses modifications peuvent encore être apportées à l'invention.

L'applicateur peut être, le cas échéant, au moins partiellement réalisé avec des fibres papetières, dans un tissé ou un non-tissé, imprégné le cas échéant d'un matériau polymérique compatible avec le produit P. La lame souple peut encore être réalisée dans un métal, notamment un matériau présentant une mémoire de forme, voire dans une mousse de faible porosité ou recouverte d'une peau du côté de la face applicatrice ou dans un papier ou carton, éventuellement pelliculé. La lame peut comporter, le cas échéant, une armature, par exemple une armature métallique ou un tissé ou des fibres synthétiques de renfort.

Dans des variantes de mise en oeuvre de l'invention, un applicateur réalisé conformément à l'invention est utilisé de manière à réaliser un maquillage dit French manucure, dans lequel une composition de couleur blanche est déposée le long du bord libre de l'ongle.

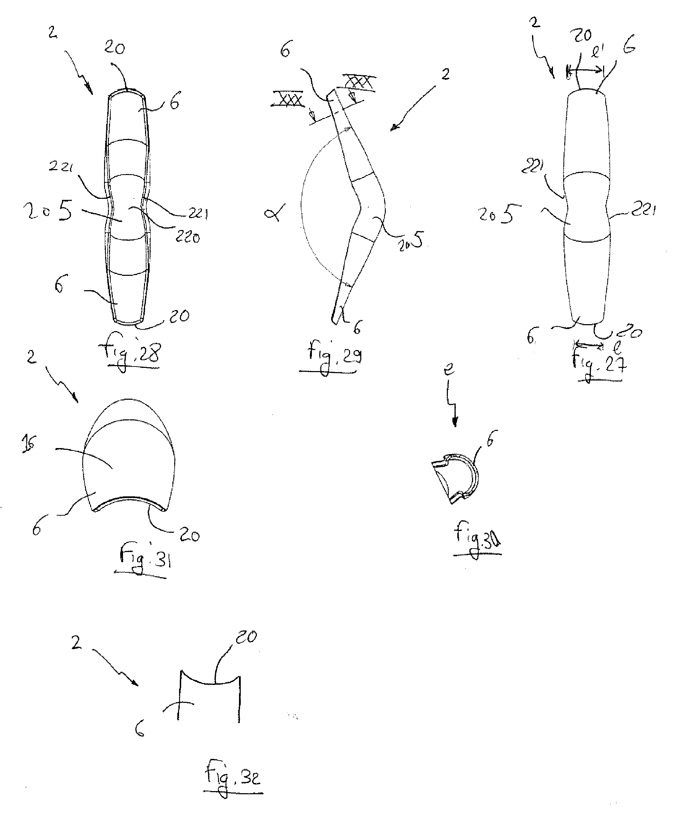

A cette fin, l'un quelconque des applicateurs décrits ci-dessus peut être modifié de manière à présenter, lorsqu'observé de dessus, un bord libre concave, comme illustré à la figure 32. La lame peut définir une surface d'application développable.

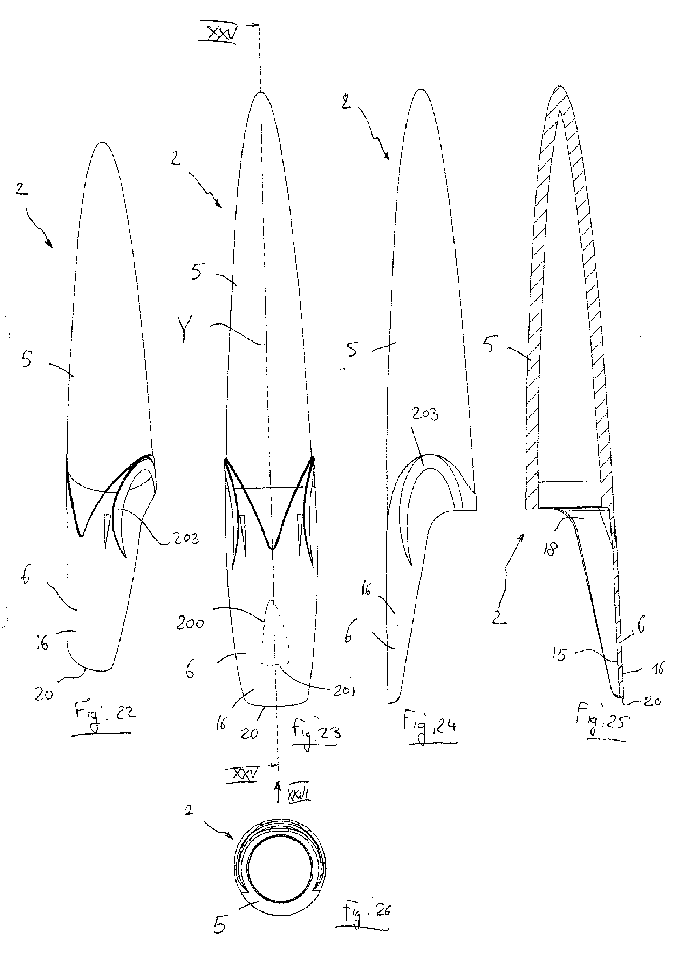

Dans la variante illustrée aux figures 22 à 26, la lame 6 est réalisée d'une seule pièce avec le manche creux 5 par moulage d'une matière thermoplastique élastomère. Il s'agit par exemple de Santoprène® 82 8135med de la société EXXON MOBIL, qui est un mélange de SBS-SEBS de dureté Shore 35 ShoreA ou de Engage® 8137 de la société SAFIC ALCAN DOW qui est un éthylène octène de dureté Shore 57 ShoreA.

Le cas échéant, comme illustré à la figure 23, la lame souple 6 peut être réalisée avec un évidement 200, lequel peut accroître la flexibilité de la lame et notamment lui permettre de se déformer à l'application lorsque l'utilisateur appuie avec son bord distal contre l'ongle.

L'évidement 200 peut présenter, par exemple, une forme généralement triangulaire, la base 201 du triangle pouvant être rectiligne ou curviligne, par exemple circulaire lorsque le bord distal de la lame est circulaire, de même centre de courbure éventuellement.

La lame 6 peut être réalisée avec une épaisseur de matière moindre que le manche 5, comme on le voit notamment sur la figure 25, le manche 5 étant par exemple réalisé avec une épaisseur de matière au moins double de celle de la lame, l'épaisseur de matière de la lame étant par exemple inférieure ou égale à 1 mm, par exemple de l'ordre de 0,5 mm tandis que celle du manche est supérieure ou égale à 1 mm.

Des nervures décoratives 203 peuvent être réalisées sur le manche comme illustré sur les figures 22 à 24.

La face applicatrice de la lame peut définir une surface d'application développable, par exemple cylindrique.

Dans l'exemple des figures 28 à 31, l'applicateur comporte deux lames souples 6 à chacune de ses extrémités, ces lames souples étant de dimensions différentes et/ou de profils différents de manière à pouvoir réaliser deux maquillages différents et/ou maquiller deux ongles de tailles différentes.

Dans l'exemple considéré, l'une des lames présente un bord distal 20 qui est plus court et de plus faible rayon de courbure que celui de l'autre lame, de façon à pouvoir appliquer la composition sur des ongles plus petits. Les deux lames souples 6 sont réunies par une partie de liaison 205 qui peut servir de moyen de préhension, l'utilisateur pouvant lors de l'application saisir aussi la lame souple non utilisée pour s'en servir comme d'un manche.

La partie de liaison 205 peut présenter une certaine déformabilité qui autorise l'utilisateur à modifier l'angle α entre le dos des lames souples 6, l'angle α étant par exemple de 130˚ environ au repos.

Le rayon de courbure de la plus petite lame souple 6 dans le plan de coupe de la figure 30, mesuré sur la surface extérieure de la lame, est par exemple compris entre 6 et 7 mm, l'épaisseur de la lame 6 étant par exemple de 0,7 mm environ.

La largeur /, mesurée en vue de dessus, du bord distal de l'une des lames est par exemple comprise entre 8 et 9 mm et la largeur /' de l'autre bord distal est par exemple comprise entre 10 et 11 mm.

La partie de liaison 205 est par exemple formée avec un dos 220 et deux parois latérales 221, comme on peut le voir sur la figure 28, les parois 221 étant infléchies l'une de l'autre dans une région médiane.

L'expression « comportant un » doit être comprise comme étant synonyme de « comportant au moins un » et « compris entre » sous-entend que les bornes sont incluses.

## Procédé

L'invention a en outre pour objet un procédé cosmétique de maquillage des ongles consistant à abaisser la viscosité d'une composition de vernis à ongles présentant une viscosité à 25˚C d'au moins 0,6 Pa.s à l'aide d'un applicateur tel que défini plus haut ou d'une action non chimique, simultanément ou préalablement à l'application sur les ongles de ladite composition.

Selon un mode de réalisation, le procédé consiste à appliquer à la composition sous forme de gel conditionné dans le récipient, une action non chimique, notamment une contrainte mécanique à l'aide d'un applicateur tel que défini plus haut, de manière à fluidifier et réduire la viscosité de ladite composition et permettre son application sur les ongles. Lorsque l'application cesse, la composition dans son conditionnement, après un temps de repos, recouvre sa texture initiale de gel.

Selon un autre mode de réalisation, le procédé consiste à prélever un échantillon de la composition dans son conditionnement, puis à appliquer audit échantillon l'action non chimique, notamment une contrainte mécanique à l'aide d'un applicateur tel que défini plus haut, de manière à fluidifier ladite composition simultanément à son application sur les ongles.

L'action non chimique peut être choisie parmi les actions thermiques telles que par exemple une source de chaleur, les actions mécaniques telles qu'un objet via lequel on applique une contrainte mécanique ou cisaillement à la composition, et leurs associations. En particulier, cet objet peut être un applicateur se présentant sous forme d'un pinceau, d'une spatule ou d'un embout, et notamment un applicateur tel que décrit ci-dessus.

De préférence, l'action non chimique est une action mécanique.

La composition de vernis à ongle de l'invention peut être conditionnée dans un récipient délimitant au moins un compartiment, ledit récipient étant fermé par un élément de fermeture.

Le récipient peut être sous toute forme adéquate et peut être, au moins pour partie, en un matériau tel que le verre. Toutefois, des matériaux autres que le verre peuvent être utilisés comme les matériaux thermoplastiques tels que le PP ou le PE ou comme un métal.

L'invention est illustrée plus en détail dans les exemples suivants. Sauf indication contraire, les quantités sont données en pourcentage en poids par rapport au poids total de la composition.

### Exemples 1 : Vernis à ongles coloré

On prépare un vernis à ongles ayant la composition suivante (% en poids) :

|  | Exemple 1 |
|---|---|
| Nitrocellulose à 30 d'alcool isopropylique (viscosité: E22 - 1/2 S) | 5,55 |
| Nitrocellulose à 30 % d'alcool isopropylique (IDYL E27 de Bergerac) | 12,12 |
| Nitrocellulose à 30 % d'alcool isopropylique (Azur E80 de Bergerac) | 0,08 |
| Résine alkyde glycérophtalique ésterifiée par acides gras ramifiés dans l'acétate d'éthyle à 70% (Beckosol ODE 230 70E de Dainippon Ink and Chemicals) | 15,50 |
| Alcool isopropylique | 1,14 |
| Cyclopentadiméthylsiloxane (DC245 fluid de Dow Corning) | 2 |
| Polydiméthylsiloxane 5 cSt (DC200 fluid de Dow Corning) | 0,44 |
| Hectorite modifiée stéaryl benzyl diméthyl ammonium (BENTONE 27 V d'Elementis) | 2,56 |
| Silice pyrogénée hydrophile (Aérosil 200 de Degussa) | 0,46 |
| Laque Red 7 | 0,02 |
| Mica oxyde de titane (Timiron Super Silk MP1005 de Merck) | 0,55 |
| Mica oxyde de titane (Flamenco Red 420 C de Engelhard) | 0,2 |
| Oxychlorure de bismuth | 0,78 |
| Acétate d'éthyle | 19,27 |
| Acétyl citrate de tributyle | 7,54 |
| Acétate de butyle | Qsp 100 |
| Acide citrique, 1 H2O | 0,1 |

La composition présente une viscosité à 25°C de 0,820 Pa.s et est conditionnée dans un pot.
On applique à la composition une agitation mécanique au moyen d'un applicateur comportant une lame souple définissant une face applicatrice, telle que décrit dans la figure 6, la composition se fluidifie puis on applique au moyen de l'applicateur la composition fluidifiée sur les ongles. On obtient un film brillant, bien couvrant sur les ongles.
Au bout de quelques minutes, le vernis à ongles retrouve sa texture initiale (viscosité proche de la viscosité initiale).

### Exemples 2 : Vernis à ongles non coloré

|  | Exemple 2 |
|---|---|
| Nitrocellulose à 30 d'alcool isopropylique (viscosité: E22 - 1/2 S) | 5,2 |
| Nitrocellulose à 30 % d'alcool isopropylique (IDYL E27 de Bergerac) | 13,70 |
| Résine alkyde glycérophtalique ésterifiée par acides gras ramifiés dans l'acétate d'éthyle à 70% (Beckosol ODE | 16,19 |

(suite)

| | Exemple 2 |
|---|---|
| 230 70E de Dainippon Ink and Chemicals). | |
| Alcool isopropylique | 0,99 |
| Polydiméthylsiloxane 5 cSt (DC200 fluid de Dow Corning) | 0,5 |
| Hectorite modifiée stéaryl benzyl diméthyl ammonium (BENTONE 27 V d'Elementis) | 2,8 |
| Silice pyrogénée hydrophile (Aérosil 200 de Degussa) | 0,526 |
| Acétate d'éthyle | 19,94 |
| Acétyl citrate de tributyle | 7,96 |
| Acétate de butyle | Qsp 100 |
| Acide citrique, 1 H2O | 0,1 |

**Exemples 3 : Vernis à ongles**

a) **Synthèse d'un polycondensat pentaérythrityl benzoate/isophtalate/isostéarate**

Dans un réacteur équipé d'une agitation mécanique, d'une arrivée d'argon et d'un système de distillation, on charge 227,5 g d'acide benzoïque, 72,8 g d'acide isostéarique et 118,3 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 18 heures. On refroidit à une température comprise entre 100 et 130°C puis on introduit 91 g d'acide isophtalique et on chauffe à nouveau progressivement jusqu'à 220°C pendant environ 11 heures.
On obtient ainsi 430 g de polycondensat pentaérythrityl benzoate/isophta-late/isostéarate sous la forme d'une huile épaisse qui se solidifie à température ambiante.
Le polycondensat présente les caractéristiques suivantes :

- Indice d'acide = 12,7
- Indice d'hydroxyle = 49
- $\eta_{110°C}$ = 25,4 Poises (soit 2540 mPa.s)
- rapport entre le nombre de mole d'acide monocarboxylique aromatique et le nombre de mole d'acide monocarboxylique non aromatique : 7,28.

On prélève 420g de polycondensat obtenu ci-dessus, on le chauffe à 100-120°C et on coule lentement 180g d'acétate de butyle sous agitation puis on clarifie par filtration à chaud sur fritté n°2.
On obtient après refroidissement à température ambiante 600 g de solution de polycondensat à 70% dans l'acétate de butyle, se présentant sous la forme d'un liquide visqueux jaune pâle et possédant une viscosité à 25°C d'environ 800 centipoises (mPa.s)

b) on prépare le vernis à ongles suivant :

| | |
|---|---|
| Nitrocellulose à 30 d'alcool isopropylique (viscosité: E22 - 1/2 S) | 4,84 |
| Nitrocellulose à 30 % d'alcool isopropylique (IDYL E27 de Bergerac) | 12 |
| Nitrocellulose à 30 % d'alcool isopropylique (Azur E80 de Bergerac) | 0,08 |

EP 2 025 320 A1

(suite)

| | |
|---|---|
| Résine alkyde glycérophtalique ésterifiée par acides gras ramifiés dans l'acétate d'éthyle à 70% (Beckosol ODE 230 70E de Dainippon Ink and Chemicals) | 2,45 |
| Solution à 70% en matières sèches du polymère synthétisé au a) dans l'acétate de butyle | 11,49 |
| Alcool isopropylique | 1,4 |
| Cyclopentadiméthylsiloxane (DC245 fluid de Dow Corning) | 2 |
| Polydiméthylsiloxane 5 cSt (DC200 fluid de Dow Corning) | 0,44 |
| Hectorite modifiée stéaryl benzyl diméthyl ammonium (BENTONE 27 V d'Elementis) | 3,19 |
| Silice pyrogénée hydrophile (Aérosil 200 de Degussa) | 0,37 |
| Laque Red 7 | 0,02 |
| Mica oxyde de titane (Timiron Super Silk MP1005 de Merck) | 0,55 |
| Mica oxyde de titane (Flamenco Red 420 C de Engelhard) | 0,2 |
| Oxychlorure de bismuth | 0,78 |
| Acétate d'éthyle | 15 |
| Acétyl citrate de tributyle | 7,37 |
| Acétate de butyle | Qsp 100 |
| Acide citrique, 1 H2O | 0,13 |

La composition présente une viscosité à 25˚C, mesurée au Rhéomat 180, de 0,820 Pa.s

On applique à la composition une agitation mécanique au moyen d'un applicateur tel que défini dans la présente demande, la composition se fluidifie puis on applique au moyen de l'applicateur la composition fluidifiée sur les ongles. On obtient un film brillant, bien couvrant sur les ongles, sans stries.

Au bout de quelques minutes, le vernis à ongles retrouve sa texture initiale (viscosité proche de la viscosité initiale).

On évalue le comportement thixotrope de la composition via les mesures de viscosité de la composition selon le protocole décrit plus haut.

La composition présente une viscosité, telle que mesurée à l'étape e), de 45 Pa.s.

Elle présente un comportement thixotrope caractérisé par la différence (viscosité mesurée au cours de l'étape c) à une vitesse de cisaillement de 1 s$^{-1}$ - viscosité mesurée au cours de l'étape e) à une vitesse de cisaillement de 1 s$^{-1}$) de l'ordre de 100 Pa.s.

Cette composition présente un module de rigidité plateau Gp compris entre 1000 et 3000 Pa, de l'ordre de 2000 Pa, une élasticité $\delta_p$ allant de 20˚, et un seuil d'écoulement $\tau_c$ de 100 Pa.

c) On prépare :

- un vernis à ongles de texture gélifiée (exemple 4 selon l'invention) présentant une viscosité, mesurée selon le protocole indiqué dans la description, supérieure à 0,6 Pa.s et
- un vernis à ongles sous forme liquide selon l'art antérieur (exemple 5 comparatif) dont la viscosité n'est pas mesurable selon le protocole indiqué plus haut.

Les vernis ont la composition suivante (% en poids) :

| | Exemple 4 (invention) | Exemple 5 (comparatif) |
|---|---|---|
| Solution à 70% en matières sèches du polymère de l'exemple 1 dans l'acétate de butyle | 12,18 | - |
| Nitrocellulose à 30 d'alcool isopropylique (viscosité: E22 - 1/2 S) | 4,18 | 12,37 |
| Nitrocellulose à 30 % d'alcool isopropylique (IDYL E27 de Bergerac) | 12,7 | - |
| Nitrocellulose à 30% d'alcool isopropylique (viscosité: E28 - 1/8 S) | - | - |
| Nitrocellulose à 30 % d'alcool isopropylique (Azur E80 de Bergerac) | 0,004 | 1,63 |
| Résine alkyde glycérophtalique esterifiée par acides gras ramifiés dans l'acétate d'éthyle à 70% (Beckosol ODE 230 70E de Dainippon Ink and Chemicals) | 1,42 | 15,53 |
| Cyclopentadiméthylsiloxane (DC245 fluid de Dow Corning) | 2 | - |
| Polydiméthylsiloxane 5 cSt (DC200 fluid de Dow Corning) | 0,47 | - |
| Hectorite modifiée stéaryl benzyl diméthyl ammonium (BENTONE 27 V d'Elementis) | 3,3 | 1,23 |
| Silice pyrogénée hydrophile (Aérosil 200 de Degussa) | 0,4 | - |
| Particules de verre enrobées d'oxyde de titane à (Metashine MC1040RY de Nippon Sheet Glass) | 1,5 | 1,5 |
| Particules de verre enrobées d'oxyde de titane (Metashine MC1040RS de Nippon Sheet Glass) | 1,5 | 1,5 |
| Mica oxyde de titane (Timiron Super Gold de Merck) | 0,8 | 0,8 |
| Laque Red 34 | 0,0045 | 0,0045 |
| Laque Red 6 | 0,005 | 0,005 |
| Bleu ferrique | 0,00009 | 0,00009 |
| Alcool isopropylique | 1,23 | 3,58 |
| Acétate d'éthyle | 14,27 | 22,51 |
| Acétyl citrate de tributyle | 7,31 | 1,73 |
| Acétate de butyle | Qsp 100 | Qsp 100 |
| N-ethyl O,P-toluenesulfonamide | - | 4,85 |
| Acide citrique, 1 H2O | 0,13 | 0,05 |

La stabilité des formules dans le temps peut être évaluée en plaçant un flacon de chaque formule dans une étuve à 45°C pendant 2 mois, puis en observant visuellement l'évolution de l'homogénéité de la formule dans le temps. L'observateur attribue une note allant de 0 à 5 après 2 mois à 45°C, (0 = sédimentation maximale ; 5 = aucune sédimentation).

La stabilité du vernis à ongles selon l'invention n'a pas varié après 2 mois à 45°C (pas de sédimentation des pigments), tandis que le vernis à ongles conventionnel sous forme liquide présente une sédimentation des pigments au fond du flacon et une couleur non homogène.

**Revendications**

1. Ensemble (1) de conditionnement et d'application comportant :

   - un récipient (3) contenant une composition de vernis à ongles comprenant un milieu cosmétiquement acceptable et au moins un agent épaississant thixotrope, ladite composition présentant une viscosité à 25°C d'au moins 0,6 Pa.s,
   - un applicateur (2) pour prélever le produit contenu dans le récipient et l'appliquer sur les ongles, comportant une lame souple (6) définissant une face applicatrice (15).

2. Ensemble selon la revendication 1, **caractérisé en ce que** la composition présente une viscosité allant de 0,6 à 20 Pa.s, de préférence de 0,7 à 15 Pa.s et, mieux de 0,75 à 10 Pa.s.

3. Ensemble (1) de conditionnement et d'application comportant :

   - un récipient (3) contenant une composition de vernis à ongles comprenant un milieu cosmétiquement acceptable et au moins un agent épaississant thixotrope, ladite composition présentant un caractère thixotrope tel que la différence de viscosité, mesurée selon le protocole indiqué dans la description, à une vitesse de cisaillement de 1 s$^{-1}$ : (viscosité mesurée au cours de l'étape c) - viscosité mesurée au cours de l'étape e)) est supérieure ou égale à 1 Pa.s.
   - un applicateur (2) pour prélever le produit contenu dans le récipient et l'appliquer sur les ongles, comportant une lame souple (6) définissant une face applicatrice (15).

4. Ensemble selon la revendication 3, **caractérisé en ce que** la différence de viscosité (viscosité mesurée au cours de l'étape c) - viscosité mesurée au cours de l'étape e)) est supérieure ou égale à 10 Pa.s, mieux d'au moins 20 Pa.s, encore mieux d'au moins 30 Pa.s, préférentiellement d'au moins 40 Pa.s.

5. Ensemble selon la revendication 3 ou 4, **caractérisée en ce que** la différence de viscosité (viscosité mesurée au cours de l'étape c) - viscosité mesurée au cours de l'étape e)) va de 1 à 1000 Pa.s, mieux de 20 à 500 Pa.s, en particulier de 40 à 200 Pa.s.

6. Ensemble (1) de conditionnement et d'application comportant :

   - un récipient (3) contenant une composition de vernis à ongles comprenant un milieu cosmétiquement acceptable et au moins un agent épaississant thixotrope, ladite composition présentant un module de rigidité plateau Gp supérieur à 100 Pa,
   - un applicateur (2) pour prélever le produit contenu dans le récipient et l'appliquer sur les ongles, comportant une lame souple (6) définissant une face applicatrice (15).

7. Ensemble selon la revendication 6, **caractérisée en ce que** le module de rigidité plateau Gp va de 100 à $2.10^6$ Pa.s, de préférence de $5.10^2$ à $10^4$ Pa.s, mieux de 800 à 4000 Pa.s, et en particulier de 1000 à 3000 Pa.s.

8. Ensemble selon l'une des revendications précédentes, **caractérisée en ce que** l'agent épaississant thixotrope est choisi parmi les argiles hydrophiles ou organophiles, les silices pyrogénées hydrophiles ou hydrophobes, les organopolysiloxanes élastomériques et leurs mélanges.

9. Ensemble selon l'une quelconque des revendications précédentes, **caractérisée en ce que** l'agent épaississant thixotrope est choisi parmi argiles modifiées organophiles telles que l'hectorite modifiée par le stéarate de benzyl di methyl ammonium.

10. Ensemble selon l'une quelconque des revendications précédentes, **caractérisée en ce que** l'agent épaississant thixotrope comprend en outre une silice pyrogénée hydrophobe.

11. Ensemble selon l'une quelconque des revendications précédentes, **caractérisée en ce que** l'agent épaississant thixotrope est présent en une teneur allant de 1,7 % à 15 % en poids, par rapport au poids total de la composition, de préférence allant de 2 % à 10% en poids, préférentiellement allant de 2 % à 7,5 % en poids, préférentiellement allant de 3% à 7,5% en poids, préférentiellement allant de 3,5% à 7,5% en poids.

**12.** Ensemble selon l'une des revendications précédentes, la lame souple (6) étant incurvée et définissant une face applicatrice (15) généralement concave.

**13.** Ensemble selon l'une quelconque des revendications précédentes, la lame souple définissant des surfaces d'appui opposées pour les doigts, de manière à permettre à l'utilisateur d'en modifier la courbure.

**14.** Ensemble selon l'une quelconque des revendications précédentes, la lame souple présentant une épaisseur non constante.

**15.** Ensemble selon la revendication 14, la lame comportant un dos situé entre des ailes plus épaisses que le dos.

**16.** Ensemble selon l'une des revendications 1 à 11, la lame souple présentant un bord distal (20) convexe lorsqu'observée de dessus.

**17.** Ensemble selon l'une quelconque des revendications précédentes, l'applicateur comportant un organe de réglage de la courbure de la face applicatrice (15).

**18.** Ensemble selon l'une quelconque des revendications précédentes, **caractérisé en ce que** la composition comprend au moins un agent de coloration choisi parmi les corps magnétiques, les pigments diffractants, les pigments interférentiels, les particules réfléchissantes et leurs mélanges, ledit agent de coloration étant présent en une teneur supérieure ou égale à 2% en poids par rapport au poids total de la composition.

**19.** Composition selon la revendication 18, **caractérisée en ce que** les pigments diffractants sont choisis parmi :

    - les pigments monocouche comprenant un matériau réfléchissant choisi parmi les métaux et leurs alliages,
    - les pigments présentant une structure multicouche comportant une couche d'un matériau réfléchissant choisi parmi les métaux et leurs alliages et aussi parmi les matériaux réfléchissants non métalliques, recouverte au moins d'un côté d'une couche d'un matériau diélectrique.
    - les pigments composés d'un matériau diélectrique ou céramique préformé tel qu'un minéral en lamelle naturelle, ou des lamelles synthétiques,

    et leurs mélanges.

**20.** Composition selon la revendication 18, **caractérisé en ce que** les pigments interférentiels sont choisis parmi les nacres, les particules réfléchissantes interférentielles, les pigments goniochromatiques et leurs mélanges.

**21.** Composition selon la revendication 18, **caractérisée en ce que** les particules réfléchissantes sont choisies parmi :

    - les oxydes métalliques, notamment les oxydes de titane ou de fer obtenus par synthèse,
    - les structures multicouches comprenant un substrat naturel ou synthétique, au moins partiellement enrobé par au moins une couche d'un matériau réfléchissant notamment d'au moins un métal ou matériau métallique.

**22.** Composition selon la revendication 18, **caractérisée en ce que** fait que les corps ou particules magnétiques comportent un matériau magnétique choisi dans le groupe constitué par : le fer, le nickel, le cobalt, leurs alliages et oxydes, notamment $Fe_3O_4$.

**23.** Composition selon l'une des revendications 18 à 22, **caractérisée en ce que** l'agent de coloration est présent en une teneur supérieure ou égale à 2,5% en poids, de préférence supérieure ou égale à 3% en poids, mieux, supérieure ou égale à 3,5% en poids, et encore mieux, supérieure ou égale à 4% en poids par rapport au poids total de la composition.

**24.** Procédé de maquillage des ongles, comportant les étapes suivantes :

    - prélever un vernis à ongles présentant une viscosité à 25°C d'au moins 0,6 Pa.s dans un récipient en utilisant la lame (6) d'un applicateur tel que défini dans l'une quelconque des revendications précédentes,
    - appliquer le produit en utilisant la face applicatrice (15) de la lame.

**25.** Procédé cosmétique de maquillage des ongles consistant à abaisser la viscosité d'une composition de vernis à ongles présentant une viscosité à 25˚C d'au moins 0,6 Pa.s à l'aide d'un applicateur tel que défini dans l'une quelconque des revendications précédentes, simultanément ou préalablement à l'application sur les ongles de ladite composition.

26. Procédé selon la revendication 24 ou 25, dans lequel avant ou après le prélèvement du produit, l'utilisateur adapte la courbure de la lame à celle de l'ongle traité.

Fig.1

Fig.2

Fig.3

Fig.4

Fig.5

Fig.10

Fig.6

Fig.8

Fig.7

Fig.9

Fig.14

Fig.12

Fig.13

Fig.11

Fig.15

Fig.16

Fig.20

Fig.21

Fig.17

Fig.19

Fig.18

Fig. 22

Fig. 23

Fig. 24

Fig. 25

Fig. 26

fig.28

fig.29

fig.27

fig.31

fig.30

fig.32

| Europäisches Patentamt European Patent Office Office européen des brevets | **RAPPORT DE RECHERCHE EUROPEENNE** | Numéro de la demande EP 08 16 1339 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,P | EP 1 818 042 A (OREAL [FR]) 15 août 2007 (2007-08-15) * le document en entier * ----- | 19,24,25 | INV. A61K8/25 A61K8/26 A61K8/29 |
| X | FR 2 664 161 A (POUDRES & EXPLOSIFS STE NALE [FR]) 10 janvier 1992 (1992-01-10) * page 2, ligne 3-9,30-35 * * page 3, ligne 1-13 * * page 4, ligne 4-9 * * page 5, ligne 31-35 * * page 6, ligne 19-26; revendications 1,2 * ----- | 19,24,25 | A61K8/41 A61Q3/02 |
| X | US 5 202 114 A (OGUSU YOSHIYUKI [US] ET AL) 13 avril 1993 (1993-04-13) * colonne 1, ligne 39-42 * * colonne 3, ligne 7 - colonne 4, ligne 43 * * colonne 5, ligne 10-62 * * colonne 10, ligne 7-11; figure 2; tableaux 1,3-1,5,6 * ----- | 19,24,25 | |
| X | US 3 864 294 A (BUSCH JR FRANCIS W) 4 février 1975 (1975-02-04) * colonne 1, ligne 41 - colonne 4, ligne 29 * * colonne 5, ligne 60-67 * * colonne 6, ligne 61 - colonne 7, ligne 3; exemples II-IV * ----- | 19,24,25 | DOMAINES TECHNIQUES RECHERCHES (IPC) A61K |
| X | WO 02/45663 A (OREAL [FR]; CARRION DANUVIO [US]; FARER ALAN [US]; FRANKFURT CHRIS [US] 13 juin 2002 (2002-06-13) * page 1, ligne 10-31; exemple 2 * * page 5, ligne 28-31 * * page 7, ligne 15 - page 8, ligne 20 * ----- | 19,24,25 | |

-/--

Le présent rapport a été établi pour toutes les revendications

| Lieu de la recherche | Date d'achèvement de la recherche | Examinateur |
|---|---|---|
| Munich | 14 novembre 2008 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 08 16 1339

| | **DOCUMENTS CONSIDERES COMME PERTINENTS** | | |
|---|---|---|---|
| Catégorie | Citation du document avec indication, en cas de besoin, des parties pertinentes | Revendication concernée | CLASSEMENT DE LA DEMANDE (IPC) |
| X | DE 36 25 418 A1 (GUTBERLET HORST [DE]) 4 février 1988 (1988-02-04) * colonne 2, ligne 46-66; revendication 1 * ----- | 1,3,6 | |
| X | EP 0 651 955 A (OREAL [FR]) 10 mai 1995 (1995-05-10) * le document en entier * ----- | 1,3,6 | |
| X | US 4 940 351 A (KONOSE YOSHIOMI [JP]) 10 juillet 1990 (1990-07-10) * le document en entier * ----- | 1,3,6 | |
| X | DATABASE WPI Week 200419 Thomson Scientific, London, GB; AN 2004-197234 XP002503941 & JP 2004 065467 A (BB LAB KK) 4 mars 2004 (2004-03-04) * abrégé * ----- | 1,3,6 | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 14 novembre 2008 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 08 16 1339

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-11-2008

| Document brevet cité au rapport de recherche | | Date de publication | Membre(s) de la famille de brevet(s) | | Date de publication |
|---|---|---|---|---|---|
| EP 1818042 | A | 15-08-2007 | BR | PI0700750 A | 13-11-2007 |
| | | | FR | 2897261 A1 | 17-08-2007 |
| | | | JP | 2007217411 A | 30-08-2007 |
| FR 2664161 | A | 10-01-1992 | AUCUN | | |
| US 5202114 | A | 13-04-1993 | AUCUN | | |
| US 3864294 | A | 04-02-1975 | AR | 198005 A1 | 24-05-1974 |
| | | | AU | 475135 B2 | 12-08-1976 |
| | | | AU | 6020173 A | 13-03-1975 |
| | | | CA | 1011485 A1 | 31-05-1977 |
| | | | DE | 2345560 A1 | 09-05-1974 |
| | | | ES | 420063 A1 | 01-07-1976 |
| | | | FR | 2205557 A1 | 31-05-1974 |
| | | | GB | 1441136 A | 30-06-1976 |
| | | | IT | 998830 B | 20-02-1976 |
| | | | JP | 895953 C | 14-02-1978 |
| | | | JP | 49097830 A | 17-09-1974 |
| | | | JP | 52024537 B | 01-07-1977 |
| | | | ZA | 7305420 A | 26-03-1975 |
| WO 0245663 | A | 13-06-2002 | AU | 4309001 A | 18-06-2002 |
| | | | CA | 2397587 A1 | 13-06-2002 |
| | | | CN | 1433296 A | 30-07-2003 |
| | | | EP | 1341501 A1 | 10-09-2003 |
| | | | JP | 2004514728 T | 20-05-2004 |
| DE 3625418 | A1 | 04-02-1988 | AUCUN | | |
| EP 0651955 | A | 10-05-1995 | CA | 2134734 A1 | 06-05-1995 |
| | | | DE | 69426591 D1 | 22-02-2001 |
| | | | DE | 69426591 T2 | 31-05-2001 |
| | | | DE | 69430220 D1 | 25-04-2002 |
| | | | DE | 69430220 T2 | 18-07-2002 |
| | | | ES | 2153411 T3 | 01-03-2001 |
| | | | ES | 2171065 T3 | 16-08-2002 |
| | | | FR | 2711898 A1 | 12-05-1995 |
| | | | JP | 10295440 A | 10-11-1998 |
| | | | JP | 2977452 B2 | 15-11-1999 |
| | | | JP | 7184716 A | 25-07-1995 |
| | | | US | 5588447 A | 31-12-1996 |
| US 4940351 | A | 10-07-1990 | DE | 3924925 C1 | 29-11-1990 |
| | | | FR | 2650201 A1 | 01-02-1991 |
| | | | GB | 2234672 A | 13-02-1991 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 08 16 1339

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-11-2008

| Document brevet cité au rapport de recherche | Date de publication | Membre(s) de la famille de brevet(s) | Date de publication |
|---|---|---|---|
| JP 2004065467 A | 04-03-2004 | AUCUN | |

Pour tout renseignement concernant cette annexe : voir Journal Officiel de l'Office européen des brevets, No.12/82

EPO FORM P0460

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0295886 A
- US 5266321 A
- EP 708114 A
- US 5783657 A
- EP 898958 A
- EP 1411069 A
- WO 04028488 A
- US 6589331 B
- US 20030031870 A
- JP 09188830 A
- JP 1158450 A
- JP 1158541 A
- JP 07258460 A
- JP 05017710 A

**Littérature non-brevet citée dans la description**

- **P. COUSSOT ; J.L. GROSSIORD.** Comprendre la rhéologie - De la circulation du sang à la prise du béton. *EDP Science,* 2002, 16, 17
- **G. COUARRAZE ; J.L. GROSSIORD.** Initiation à la rhéologie. 1991
- Silica silylate. CTFA. 1995
- International Cosmetic Ingredient Dictionnary and Handbook. The Cosmetic, Toiletry, and Fragrance Association, 1997, 386, 524-528